(19) Europäisches Patentamt
European Patent Office
Office européen des brevets

(11) Publication number: **0 202 794**
**A1**

(12) **EUROPEAN PATENT APPLICATION**

(21) Application number: 86303175.3

(22) Date of filing: 28.04.86

(51) Int. Cl.[4]: C 07 D 487/04
//C07D231/08, (C07D487/04, 231:00, 231:00)

(30) Priority: 30.04.85 US 728732

(43) Date of publication of application: 26.11.86 Bulletin 86/48

(84) Designated Contracting States: AT BE CH DE FR GB IT LI NL SE

(71) Applicant: ELI LILLY AND COMPANY
Lilly Corporate Center
Indianapolis Indiana 46285(US)

(72) Inventor: Jungheim, Louis Nickolaus
5706 North Washington Boulevard
Indianapolis Indiana 46220(US)

(72) Inventor: Sigmund, Sandra Kay
5619 Fjord Drive Apt. E.
Indianapolis Indiana 46250(US)

(72) Inventor: Holmes, Richard Elmer
4848 Laurel Circle
Indianapolis Indiana 46226(US)

(72) Inventor: Barnett, Charles Jackson
7540 North Pennsylvania
Indianapolis Indiana 46240(US)

(74) Representative: Hudson, Christopher Mark et al,
Erl Wood Manor
Windlesham Surrey GU20 6PH(GB)

(54) **7-Substituted-2,3-(Dihydro) bicyclic pyrazolidinones.**

(57) 7-Substituted-2,3-(dihydro) bicyclic pyrazolidinones are intermediates for 7-substituted pyrazolidinone antimicrobials. The intermediates have the formula

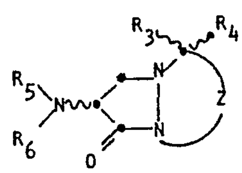

(I)

wherein Z is a group of the formula

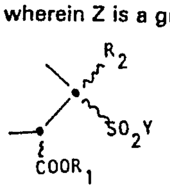

or

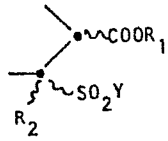

,

and wherein:

$R_1$ is hydrogen, an organic or inorganic cation, a carboxy-protecting group or a non-toxic, metabolically-labile, ester-forming group;

$R_2$ is hydrogen, halo, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano;

a group of the formula

$-CX_3$

wherein X is fluoro, chloro, bromo or iodo;

a group of the formula

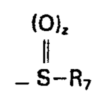

wherein z is 0, 1 or 2 and $R_7$ is $C_1$ to $C_6$ alkyl, $C_1$ to $C_6$ substituted alkyl, phenyl, substituted phenyl, $C_7$ to $C_{12}$ arylalkyl, $C_7$ to $C_{12}$ substituted arylalkyl, or a heterocyclic ring; a group of the formula

$-COR_8$

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)amino or disubstituted amino;

a group of the formula

$-COOR_9$

wherein $R_9$ is hydrogen, an organic or inorganic cation, $C_1$ to $C_6$ alkyl, $C_1$ to $C_6$ substituted alkyl, $C_7$ to $C_{12}$ arylalkyl, $C_7$ to

EP 0 202 794 A1

$C_{12}$ substituted arylalkyl, phenyl substituted phenyl, a carboxy-protecting group, or a non-toxic, metabolically-labile, ester-forming group,

or a group of the formula

$-CH_2$-S-Heterocyclic ring;

$R_3$ and $R_4$ are hydrogen or; either $R_3$ or $R_4$ is $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl or a group of the formula

$-COOR_{10}$

wherein $R_{10}$ has the same definition as $R_9$; and the other is hydrogen;

$R_5$ and $R_6$ are

1) each hydrogen; or

2) taken together and form a phthalimido group; or

3) either $R_5$ or $R_6$ is hydrogen and the other is an amino-protecting group; and Y is $C_1$ to $C_6$ alkyl, $C_1$ to $C_6$ substituted alkyl, phenyl, substituted phenyl, $C_7$ to $C_{12}$ arylalkyl or $C_7$ to $C_{12}$ substituted arylalkyl,

or a pharmaceutically acceptable salt thereof

7-SUBSTITUTED-2,3-(DIHYDRO) BICYCLIC PYRAZOLIDINONES

The invention is directed to compounds of the Formula I:

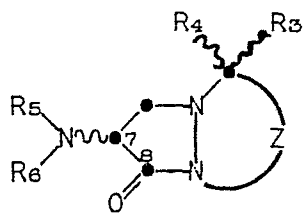

I

wherein Z is a group of the formula

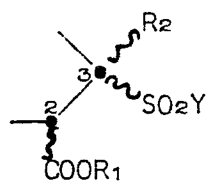

IA

or

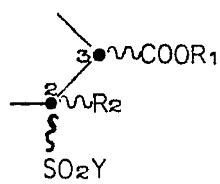

IB

The ring system of compound Formula I is a 1,5-diazabicyclo[3.3.0]octane ring, which for brevity's sake, will also be called a "saturated bicyclic pyrazolidinone" or, more simply, a "bicyclic pyrazolidinone".

The two possible 2,3-regioisomers of Formula I will be distinguished throughout the Specification by reference to the position of the sulfonyl group. Thus, the regioisomers will be referred to as either the "3-sulfonyl bicyclic pyrazolidinone" or the "3-sulfonyl regioisomer" (wherein Z in Formula I is Formula IA) and the "2-sulfonyl bicyclic pyrazolidinone" or the "2-sulfonyl regioisomer" (wherein Z in Formula I is Formula IB). The numbering system for the ring system is denoted in Formula I.

The undulating lines connecting the substituents to positions 2, 3, 4 and 7 of the bicyclic pyrazolidinone ring in Formula I indicates that the Formula embraces compounds that are independently in the R or S configuration at these positions. Furthermore, the Formula represents various percentage mixtures of the possible enantiomers and diastereomers.

In the above Formula I:

$R_1$ is hydrogen, an organic or inorganic cation, a carboxy-protecting group or a non-toxic, metabolically-labile ester-forming group;

$R_2$ is hydrogen, halo, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano;

a group of the formula

$$-CX_3$$

wherein X is fluoro, chloro, bromo or iodo;

a group of the formula

$$\overset{(O)_Z}{\overset{||}{-S-R_7}}$$

wherein Z is 0, 1 or 2 and $R_7$ is $C_1$ to $C_6$ alkyl, $C_1$ to $C_6$ substituted alkyl, phenyl, substituted phenyl, $C_7$ to $C_{12}$ arylalkyl, $C_7$ to $C_{12}$ substituted arylalkyl or a heterocyclic ring;

a group of the formula

$$-COR_8$$

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)amino, or (disubstituted)-amino;

a group of the formula

$$-COOR_9$$

wherein $R_9$ is hydrogen, an organic or inorganic cation, $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl, a carboxy protecting group or a non-toxic, metabolically-labile ester-forming group;

or

a group of the formula

$$-CH_2-S\text{-Heterocyclic ring;}$$

$R_3$ and $R_4$ are the same and are hydrogen or different and either $R_3$ or $R_4$ is hydrogen, $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl or a group of the formula

$$-COOR_{10}$$

wherein $R_{10}$ has the same definition as $R_9$; and the other is hydrogen;

$R_5$ and $R_6$ are:

1) each hydrogen;

2) taken together and form a phthalimido group;

3) either $R_5$ or $R_6$ is hydrogen and the other is an amino-protecting group;

Y is $C_1$ to $C_6$ alkyl, $C_1$ to $C_6$ substituted alkyl, phenyl, substituted phenyl, $C_7$ to $C_{12}$ arylalkyl or $C_7$ to $C_{12}$ substituted arylalkyl;

or a pharmaceutically-acceptable salt thereof.

In the above Formula I, the term "$C_1$ to $C_6$ alkyl" denotes such radicals as methyl, ethyl, n-propyl, iso-propyl, n-butyl, sec-butyl, tert-butyl, amyl, tert-amyl, hexyl and the like. The preferred "$C_1$ to $C_6$ alkyl" group is methyl.

The term "$C_1$ to $C_6$ substituted alkyl" denotes the above $C_1$ to $C_6$ alkyl groups that are substituted by one or two halo, hydroxy, protected hydroxy, amino, protected amino, $C_1$ to $C_7$ acyloxy, carboxy, protected carboxy, carbamoyl, carbamoyloxy, cyano, methylsulfonylamino or $C_1$ to $C_4$ alkoxy groups. The substituted alkyl groups may be substituted twice with the same or with different types of the above substituents.

Examples of the above substituted alkyl groups include the cyanomethyl, hydroxymethyl, trityloxymethyl, tetrahydropyranyloxymethyl, propionyloxymethyl, aminomethyl, carboxymethyl, allyloxycarbonylmethyl, allyloxycarbonylaminomethyl, carbamoyloxymethyl, methoxymethyl, ethoxymethyl, t-butoxymethyl, acetoxymethyl, chloromethyl, bromomethyl, iodomethyl, 6-hydroxyhexyl, 2,4-dichloro(n-butyl), 2-amino(iso-propyl), 2-carbamoyloxyethyl and the like. A preferred group of examples within the above "$C_1$ to $C_6$ substituted alkyl" group includes the substituted methyl group, in other words, a methyl group substituted by the same substituents as the "$C_1$ to $C_6$ substituted alkyl" group. Examples of the substituted methyl group include groups such as hydroxymethyl, protected hydroxymethyl, (e.g., tetrahydropyranyloxymethyl), acetoxymethyl, carbamoyloxymethyl, chloromethyl, bromomethyl and iodomethyl.

The term "$C_1$ to $C_4$ alkoxy" as used herein denotes groups such as methoxy, ethoxy, n-propoxy, iso-propoxy, n-butoxy, t-butoxy and like groups. Similarly, the term "$C_1$ to $C_7$ acyloxy" denotes herein groups such as formyloxy, acetoxy, propionyloxy, butyryloxy, pentanoyloxy, hexanoyloxy, heptanoyloxy, and the like.

Examples of the term "perfluoro $C_2$ to $C_4$ alkyl" include perfluoroethyl, perfluoro n-propyl, perfluoro iso-propyl, perfluoro n-butyl, perfluoro sec-butyl and the like.

The term "substituted phenyl" specifies a phenyl group substituted with one or two moieties chosen from the group consisting of halogen, hydroxy,

protected hydroxy, cyano, nitro, $C_1$ to $C_6$ alkyl, $C_1$ to $C_4$ alkoxy, carboxy, protected carboxy, carboxymethyl, protected carboxymethyl, hydroxymethyl, protected hydroxymethyl, aminomethyl, protected aminomethyl, trifluoromethyl or methylsulfonylamino.

Examples of the term "substituted phenyl" include a mono- or di(halo)phenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono- or di(alkoxy)phenyl group, for example, 2,6-dimethoxyphenyl, 4-methoxyphenyl, 3-ethoxyphenyl, 4-(iso-propoxy)phenyl, 4-(t-butoxy)phenyl, 3-ethoxy-4-methoxyphenyl and the like; 3- or 4- trifluoromethylphenyl; a mono- or dicarboxyphenyl or (protected carboxy) phenyl group such as 4-carboxyphenyl or 2,4-di(protected carboxy)phenyl, a mono- or di(hydroxymethyl)phenyl or (protected hydroxymethyl)phenyl such as 3-(protected hydroxymethyl)phenyl or 3,4-di(hydroxymethyl)phenyl, a mono- or di(aminomethyl)phenyl or (protected aminomethyl)phenyl such as 2-(aminomethyl)phenyl or 2,4-(protected aminomethyl)phenyl, or a mono- or di(methylsulfonylamino)phenyl such as 3-(methylsulfonylamino)-phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl and the like. Preferred substituted phenyl groups include the 3- and 4-trifluoromethylphenyl, the 4-hydroxyphenyl, the 2-aminomethylphenyl and the 3-(methylsulfonylamino)phenyl groups.

The terms "halo" and "halogen" refer to fluoro, chloro, bromo or iodo.

The term "trihalomethyl" denotes trifluoromethyl, trichloromethyl, tribromomethyl or triiodomethyl.

The term "$C_7$ to $C_{12}$ arylalkyl" denotes a $C_1$ to $C_6$ alkyl group substituted at any position by a phenyl ring. Examples of such a group include phenyl methyl(benzyl), 2-phenylethyl, 3-phenyl-(n-propyl), 4-phenylhexyl, 3-phenyl-(n-amyl), 3-phenyl-(sec-butyl) and the like. A preferred group is the benzyl group.

The term "$C_7$ to $C_{12}$ substituted arylalkyl" denotes a $C_7$ to $C_{12}$ substituted arylalkyl group substituted on the $C_1$ to $C_6$ alkyl portion with one or two groups chosen from halo, hydroxy, protected hydroxy, amino, protected amino, $C_1$ to $C_7$ acyloxy, carboxy, protected carboxy, carbamoyl, carbamoyloxy, cyano, methylsulfonylamino or $C_1$ to $C_4$ alkoxy; and/or the phenyl group may be substituted with 1 or 2 groups chosen from halo, hydroxy, protected hydroxy, nitro, $C_1$ to $C_6$ alkyl, $C_1$ to $C_4$ alkoxy, carboxy, protected carboxy,

carboxymethyl, protected carboxymethyl, hydroxymethyl, protected hydroxymethyl, aminomethyl, protected aminomethyl or a methylsulfonylamino group. As before, when either the $C_1$ to $C_6$ alkyl portion or the phenyl portion or both are disubstituted, the substituents can be the same or different.

Examples of the term "$C_7$ to $C_{12}$ substituted arylalkyl" include groups such as 2-phenyl-1-chloroethyl, 2-(4-methoxyphenyl)ethyl, 2,6-dihydroxy-4-phenyl(n-hexyl), 5-cyano-3-methoxy-2-phenyl(n-pentyl), 3-(2,6-dimethylphenyl)n-propyl, 4-chloro-3-aminobenzyl, 6-(4-methoxyphenyl)-3-carboxy(n-hexyl), 5-(4-aminomethyl phenyl)-3-(aminomethyl)(n-pentyl) and the like.

The term "(monosubstituted)amino" refers to amino group with one substituent chosen from the group phenyl, substituted phenyl, $C_1$ to $C_6$ alkyl, and $C_7$ to $C_{12}$ arylalkyl, wherein the latter three substituent terms are as defined above.

The term "(disubstituted)amino" refers to amino groups with two substituents chosen from the group phenyl, substituted phenyl, $C_1$ to $C_6$ arylalkyl, and $C_7$ to $C_{12}$ arylalkyl wherein the latter three substituent terms are as described above. The two substituents can be the same or different.

The term "organic or inorganic cation" refers to counter-ions for the carboxylate anion of a carboxylate salt. The counter-ions are chosen from the alkali and alkaline earth metals such as lithium, sodium, potassium, barium, and calcium; ammonium; and organic cations such as dibenzylammonium, benzylammonium,

2-hydroxyethylammonium, bis(2-hydroxyethyl)ammonium, phenylethylbenzylammonium, dibenzylethylenediammonium, and like cations. Other cations encompassed by the above term include the protonated form of procaine, quinine and N-methylglucosamine, and the protonated forms of basic amino acids such as glycine, ornithine, histidine, phenylglycine, lysine and arginine. Furthermore, any zwitterionic form of the instant compounds formed by a carboxylic acid and an amino group is referred to by this term. A preferred cation for the carboxylate anion is the sodium cation.

The term "pharmaceutically acceptable salt" encompasses those salts that form with the carboxylate anions and includes salts formed with the organic and inorganic cations discussed above. Furthermore, the term includes salts that form by standard acid-base reactions with basic groups (such as amino groups) and organic or inorganic acids. Such acids include hydrochloric, sulfuric, phosphoric, acetic, succinic, citric, lactic, maleic, fumaric, palmitic, cholic, pamoic, mucic, D-glutamic, d-camphoric, glutaric, phthalic, tartaric, lauric, stearic, salicyclic, methanesulfonic, benzenesulfonic, sorbic, picric, benzoic, cinnamic and like acids.

The compounds of Formula I may also exist as the solvates and hydrates of any pharmaceutically acceptable salts. Thus, the salts of these compounds may crystallize with, for example, waters of hydration, or one, a number of, or any fraction thereof of molecules of the mother liquor solvent. The solvates and

hydrates of such salts are included within the scope of this invention.

The term "carboxy-protecting group" as used in the specification refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl, 4-methoxytrityl, 4,4'-dimethoxytrityl, 4,4',4''-trimethoxytrityl, 2-phenylprop-2-yl, trimethylsilyl, t-butyldimethylsilyl, phenacyl, 2,2,2-trichloroethyl, β-(trimethylsilyl)ethyl, β-(di(n-butyl)methylsilyl)-ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the bicyclic pyrazolidinone molecule and can be removed at the appropriate point without disrupting the remainder of either the saturated bicyclic pyrazolidinone intermediates or the bicyclic pyrazolidinone final products. In particular, it is important not to subject the carboxy-protected bicyclic pyrazolidinone molecule to strong nucleophilic bases, or reductive conditions

employing highly activated metal catalysts such as Raney nickel. (Such harsh removal conditions are also to be avoided when removing amino-protecting groups and hydroxy-protecting groups, discussed below.) A preferred carboxylic acid protecting group is the allyl group. Similar carboxy-protecting groups used in the cephalosporin, penicillin and peptide arts can also be used to protect carboxy group substituents of the bicyclic pyrazolidinones. Further examples of these groups are found in E. Haslam, "Protective Groups in Organic Chemistry" J.G.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5. The term "protected carboxy" indicates a carboxy group substituted with one of the above carboxy-protecting groups.

The term "hydroxy-protecting group" refers to readily cleavable groups bonded to hydroxyl groups, such as the tetrahydropyranyl, 2-methoxyprop-2-yl, 1-ethoxyethyl, methoxymethyl, β-methoxyethoxymethyl, methylthiomethyl, t-butyl, t-amyl, trityl, 4-methoxytrityl, 4,4'-dimethoxytrityl, 4,4',4''-trimethoxytrityl, benzyl, allyl, trimethylsilyl, (t-butyl)dimethylsilyl, and 2,2,2-trichloroethoxycarbonyl groups and the like. The term "protected hydroxy" refers to a hydroxy group derivatized with a hydroxy-protecting group.

The species of hydroxy-protecting group is not critical so long as the derivatized hydroxyl group is stable to the conditions of subsequent reaction(s) and can be removed at the appropriate point without disrupting the remainder of either the saturated bicyclic pyrazolidinone intermediates or the bicyclic pyrazolidinone final products.

Further examples of hydroxy-protecting groups are described by C.B. Reese and E. Haslam, "Protective Groups in Organic Chemistry", J.G.W. McOmie, Ed., Plenum Press, New York, N.Y., 1973, Chapters 3 and 4, respectively, and T.W. Greene, "Protective Groups in Organic Synthesis", John Wiley and Sons, New York, N.Y., 1981, Chapters 2 and 3. Some preferred hydroxy protecting groups are the trityl group and the tetrahydropyranyl group.

The term "amino-protecting group" as used in the specification refers to substituents of the amino group commonly employed to block or protect the amino functionality while carrying out reactions at other functional groups on the compound. Examples of such amino protecting groups include the formyl group, the phthalimido group, the trichloroacetyl group, the chloroacetyl, bromoacetyl and iodoacetyl groups, urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, 2-(4-xenyl)iso-propoxycarbonyl, 1,1-diphenyleth-1-yloxycarbonyl, 1,1-diphenylprop-1-yloxycarbonyl, 2-phenylprop-2-yloxycarbonyl, 2-(p-toluyl)prop-2-yloxy-

carbonyl, cyclopentanyloxycarbonyl, 1-methylcyclopentanyloxycarbonyl, cyclohexanyloxycarbonyl, 1-methylcychexanyloxycarbonyl, 2-methylcyclohexanyloxycarbonyl, 2-(4-toluylsulfonyl)ethoxycarbonyl, 2-(methylsulfonyl)ethoxycarbonyl, 2-(triphenylphosphino)ethoxycarbonyl, 9-fluorenylmethoxycarbonyl ("FMOC"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like, the 2-benzoylmethylsulfonyl group, the 2-(nitro)phenylsulfenyl group, the diphenylphosphine oxide group and like amino-protecting groups. The species of amino-protecting group employed is not critical so long as the derivatized amino group is stable to the condition of subsequent reaction(s) on other positions of the bicyclic pyrazolidinone molecule and can be removed at the appropriate point without disrupting the remainder of the saturated bicyclic pyrazolidinone intermediate or the bicyclic pyrazolidinone final product. Preferred amino-protecting groups are the allyloxycarbonyl, the t-butoxycarbonyl, and the trityl groups. Similar amino-protecting groups used in the cephalosporin, penicillin and peptide art are also embraced by the above term. Further examples of groups referred to by the above term are described by J.W. Barton, "Protective Groups In Organic Chemistry", J.G.W. McOmie, Ed., Plenum Press, New York, N.Y., 1973, Chapter 2, and T.W. Greene, "Protective Groups in Organic Synthesis",

John Wiley and Sons, New York, N.Y., 1981, Chapter 7. Similarly, the term "protected amino" denotes an amino group derivatized with one of the above amino-protecting groups.

The term "non-toxic, metabolically-labile ester-forming group" refers to those biologically active ester forms which induce increased blood levels and prolong the efficacy of the corresponding non-esterified forms of the compounds. Such ester groups include the lower alkoxymethyl groups, for example, methoxymethyl, ethoxymethyl, iso-propoxymethyl and the like; the α-($C_1$ to $C_4$)alkoxyethyl groups, for example methoxyethyl, ethoxyethyl, propoxyethyl, iso-propoxyethyl, and the like; the 2-oxo-1,3-dioxolen-4-ylmethyl groups, such as 5-methyl-2-oxo-1,3-dioxolen-4-ylmethyl, 5-phenyl-2-oxo-1,3-dioxolen-4-ylmethyl, and the like; the $C_1$ to $C_3$ alkylthiomethyl groups, for example methylthiomethyl, ethylthiomethyl, isopropylthiomethyl, and the like; the acyloxymethyl groups, for example pivaloyloxymethyl, α-acetoxymethyl, and the like; the ethoxycarbonyl-1-methyl group; the α-acyloxy-α-substituted methyl groups, for example α-acetoxyethyl; the 3-phthalidyl or 5,6-dimethylphthalidyl groups, the 1-($C_1$ to $C_4$ alkyloxycarbonyloxy)ethyl groups such as the 1-(ethoxycarbonyloxy)ethyl group; and the 1-($C_1$ to $C_4$ alkylaminocarbonyloxy)ethyl groups such as the 1-(methylaminocarbonyloxy)ethyl group.

The term "heterocyclic ring" denotes optionally substituted five-membered or six-membered rings that have 1 to 4 heteroatoms, such as oxygen, sulfur

and/or nitrogen, in particular nitrogen, either alone or in conjunction with sulfur or oxygen ring atoms. These five-membered or six-membered rings may be fully unsaturated or partially unsaturated, with fully unsaturated rings being preferred.

Furthermore, the above optionally substituted five-membered or six-membered rings can optionally be fused to a aromatic 5-membered or 6-membered ring system. For example, the rings can be optionally fused to an aromatic 5-membered or 6-membered ring system such as pyridine or a triazole system, and preferably to a benzene ring.

The following ring systems are examples of the heterocyclic (whether substituted or unsubstituted) radicals denoted by the term "heterocyclic ring": thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, thiazinyl, oxazinyl, triazinyl, thiadiazinyl, oxadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl, tetrazolo[1,5-b]pyridazinyl and purinyl, as well as benzo-fused derivatives, for example benzoxazolyl, benzthiazolyl, benzimidazolyl and indolyl.

A preferred group of examples of the above heterocyclic rings when the heterocyclic rings form part of a heterocyclic thiomethyl group at $R_2$ include ring systems which are 5-membered ring systems containing a sulfur or oxygen atom and one to three nitrogen atoms. Examples of such preferred groups include thiazolyl, in particular thiazol-2-yl and thiazol-2-yl N-oxide, thiadiazolyl, in particular 1,3,4-thiadiazol-5-yl and 1,2,4-thiadiazol-5-yl, oxazolyl, preferably oxazol-2-yl, and oxadiazolyl, such as 1,3,4-oxadiazol-5-yl and 1,2,4-oxadiazol-5-yl. A group of further preferred examples of 5-membered ring systems with 2 to 4 nitrogen atoms include imidazolyl, preferably imidazol-2-yl, triazolyl, preferably 1,3,4-triazol-5-yl, 1,2,3-triazol-5-yl and 1,2,4-triazol-5-yl, and tetrazolyl, preferably 1H-tetrazol-5-yl. A preferred group of examples of benzo-fused derivatives are, in particular, benzoxazol-2-yl, benzthiazol-2-yl and benzimidazol-2-yl.

Further specific examples of the above heterocyclic ring systems when they are a part of a heterocyclic thiomethyl group are 6-membered ring systems containing one to three nitrogen atoms. Such examples include pyridyl, such as pyrid-2-yl, pyrid-3-yl and pyrid-4-yl, pyrimidyl, preferably pyrimid-2-yl and pyrimid-4-yl, triazinyl, preferably 1,3,4-triazin-2-yl and 1,3,5-triazin-4-yl, pyridazinyl, in particular pyridazin-3-yl, and pyrazinyl. The pyridyl, pyrimid-2-yl, pyrimid-4-yl, pyridazinyl and the 1,3,4-triazin-2-yl radicals, in addition to the pyridine N-oxides and pyridazine N-oxides, are a preferred group.

The substituents for the optionally substituted heterocyclic ring systems, and further examples of the 5- and 6- membered ring systems discussed above,

are found in W. Dürckheimer et al., U.S. Patent No. 4,278,793, issued July 14, 1981, columns 9 through 21 and columns 33 through 188, herein incorporated by reference. (In columns 33 through 188, the substituents under the heading "A" are examples of "heterocyclic ring" when the ring is a part of heterocyclic thiomethyl group).

A particularly preferred group of examples of the term "heterocyclic ring", when the ring is part of a heterocyclic thiomethyl group, is 1,3-thiazol-2-yl, 4-(carboxymethyl)-5-methyl-1,3-thiazol-2-yl, 4-(carboxymethyl)-5-methyl-1,3-thiazol-2-yl sodium salt, 1,2,4-thiadiazol-5-yl, 3-methyl-1,2,4-thiadiazol-5-yl, 1,3,4-triazol-5-yl, 2-methyl-1,3,4-triazol-5-yl, 2-hydroxy-1,3,4-triazol-5-yl, 2-(carboxy)-4-methyl-1,3,4-triazol-5-yl sodium salt, 2-(carboxy)-4-methyl-1,3,4-triazol-5-yl, 1,3-oxazol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-methyl-1,3,4-thiadiazol-5-yl, 2-thiol-1,3,4-thiadiazol-5-yl, 2-(methylthio)-1,3,4-thiadiazol-5-yl, 2-amino-1,3,4-thiadiazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 2-methyl-1H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(N-oxide)-pyridaz-3-yl,

6-hydroxypyridaz-3-yl, 1-methylpyrid-2-yl, 1-methyl-pyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-as-triazin-3-yl, 2,5-dihydro-5-oxo-6-hydroxy-as-triazin-3-yl sodium salt, 2,5-dihydro-5-oxo-6-hydroxy-2-methyl-as-triazin-3-yl sodium salt, 2,5-dihydro-5-oxo-6-hydroxy-2-methyl-as-triazin-3-yl, 2,5-dihydro-5-oxo-6-methoxy-2-methyl-as-triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5-b]-pyridazin-6-yl, and 8-aminotetrazolo[1,5-b]pyridazin-6-yl.

A most preferred group of examples of the term "heterocyclic ring" when the term is used in conjunction with a heterocyclic thiomethyl group are 4-(carboxymethyl)-5-methyl-1,3-thiazol-2-yl, 4-(carboxymethyl)-5-methyl-1,3-thiazol-2-yl sodium salt, 1,3,4-triazol-5-yl, 2-methyl-1,3,4-triazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methyl-sulfonic acid)-1H-tetrazol-5-yl, 1-(methylsulfonic acid)-1H-tetrazol-5-yl sodium salt, 1,2,3-triazol-5-yl, 1,4,5,6-tetrahydro-5,6-dioxo-4-methyl-as-triazin-3-yl, 1,4,5,6-tetrahydro-4-(2-formylmethyl)-5,6-dioxo-as-triazin-3-yl, 2,5-dihydro-5-oxo-6-hydroxy-2-methyl-as-triazin-3-yl sodium salt, 2,5-dihydro-5-oxo-6-hydroxy-2-methyl-as-triazin-3-yl, tetrazolo[1,5-b]pyridazin-6-yl, and 8-aminotetrazolo[1,5-b]pyridazin-6-yl.

In the above Formula I, when $R_2$ is a group of the formula

$$-\overset{(O)_z}{\overset{\|}{S}}-R_7$$

wherein $R_7$ is a heterocyclic group, examples of such groups are 1,3-thiazol-2-ylthio, 4-(carboxymethyl)-5-methyl-1,3-thiazol-2-ylthio, 4-(carboxymethyl)-5-methyl-1,3-thiazol-2-ylthio sodium salt, 1,2,4-thiadiazol-5-ylthio, 3-methyl-1,2,4-thiadiazol-5-ylthio, 1,3,4-triazol-5-ylthio, 2-methyl-1,3,4-triazol-5-ylthio, 2-hydroxy-1,3,4-triazol-5-ylthio, 2-(carboxy)-4-methyl-1,3,4-triazol-5-ylthio sodium salt, 2-(carboxy)-4-methyl-1,3,4-triazol-5-ylthio, 1,3-oxazol-2-ylthio, 1,3,4-oxadiazol-5-ylthio, 2-methyl-1,3,4-oxadiazol-5-ylthio, 2-(hydroxymethyl)-1,3,4-oxadiazol-5-ylthio, 1,2,4-oxadiazol-5-ylthio, 1,2,4-oxadiazol-5-ylthio, 1,3,4-thiadiazol-5-ylthio, 2-methyl-1,3,4-thiadiazol-5-ylthio, 2-thiol-1,3,4-thiadiazol-5-ylthio, 2-(methylthio)-1,3,4-thiadiazol-5-ylthio, 2-amino-1,3,4-thiadiazol-5-ylthio, 1H-tetrazol-5-ylthio, 1-methyl-1H-tetrazol-5-ylthio, 1-(1-(dimethylamino)eth-2-ylthio)-1H-tetrazol-5-ylthio, 1-(carboxymethyl)-1H-tetrazol-5-ylthio, 1-(carboxymethyl)-1H-tetrazol-5-ylthio sodium salt, 1-(methylsulfonic acid)-1H-tetrazol-5-ylthio, 1-(methylsulfonic acid)-1H-tetrazol-5-ylthio sodium salt, 2-methyl-1H-tetrazol-5-ylthio, 1,2,3-triazol-5-ylthio, 1-methyl-1,2,3-triazol-5-ylthio, 2-methyl-1,2,3-triazol-5-ylthio, 4-methyl-1,2,3-triazol-5-ylthio, pyrid-2-ylthio N-oxide, 6-methoxy-2-(N-oxide)-pyridaz-3-ylthio, 6-hydroxypyridaz-3-ylthio, 1-methylpyrid-

2-ylthio, 1-methylpyrid-4-ylthio, 2-hydroxypyrimid-4-ylthio, 1,4,5,6-tetrahydro-5,6-dioxo-4-methyl-as-triazin-3-ylthio, 1,4,5,6-tetrahydro-4-(2-formylmethyl)-5,6-dioxo-as-triazin-3-ylthio, 2,5-dihydro-5-oxo-6-hydroxy-as-triazin-3-ylthio, 2,5-dihydro-5-oxo-6-hydroxy-as-triazin-3-ylthio sodium salt, 2,5-dihydro-5-oxo-6-hydroxy-2-methyl-as-triazin-3-ylthio sodium salt, 2,5-dihydro-5-oxo-6-hydroxy-2-methyl-as-triazin-3-ylthio, 2,5-dihydro-5-oxo-6-methoxy-2-methyl-as-triazin-3-ylthio, 2,5-dihydro-5-oxo-as-triazin-3-ylthio, 2,5-dihydro-5-oxo-2-methyl-as-triazin-3-ylthio, 2,5-dihydro-5-oxo-2,6-dimethyl-as-triazin-3-ylthio, tetrazolo[1,5-b]pyridazin-6-ylthio, and 8-aminotetrazolo-[1,5-b]pyridazin-6-ylthio; the corresponding sulfoxides and sulfones of the above heterocyclic thio groups, and the like.

Examples of the above group when $R_7$ is other than a heterocyclic group include $C_1$ to $C_6$ alkylthio groups such as methylthio, ethylthio, (sec-butyl)thio, (t-amyl)thio and (n-hexyl)thio, $C_7$ to $C_{12}$ alkylphenylthio groups such as (2-phenyl)propylthio, benzylthio, 1-phenyl(n-amyl)thio and 4-phenyl(n-butyl)thio; $C_1$ to $C_6$ substituted alkylthio groups such as cyanomethylthio, 2-hydroxyethylthio, 2-nitropropylthio, 2-carbamoyl(sec-butyl)thio, 5-chloroamylthio, 4-carboxyamylthio, 6-carbamoyloxyhexylthio, 2-methoxyethylthio, isopropoxy-(t-butyl)thio, 2-aminoethylthio, 2,5-dihydroxyamylthio, 3,3-dibromo(n-butyl)thio, 3-chloro-2-iodopropylthio and 4-acetoxy-6-fluorohexylthio; $C_7$ to $C_{12}$ substituted phenylalkylthio groups such as 3-(3,4-diiodophenyl)-propylthio, 1-(3-chloro-4-fluorophenyl)ethylthio, 6-(4-cyanophenyl)hexylthio, 3-phenyl-1-chloro(sec-butyl)thio, 2-phenyl-2-hydroxyethylthio, 5-phenyl-2-hydroxyamylthio, 2-(3-nitrophenyl)-3-ethoxypropylthio, 5,6-dihydroxy-2-(4-ethyl-2-hydroxyphenyl)hexylthio and 5-carbamoyl-3-nitro-4-(2,4-dimethoxyphenyl)amylthio; phenylthio, and (substituted phenyl)thio groups, and the corresponding sulfoxide and sulfone analogs thereof.

Examples of the (substituted phenyl)thio groups represented by $R_7$ include groups such as 4-chlorophenylthio, 2,6-dichlorophenylthio, 2,5-dichlorophenylthio, 3,4-dichlorophenylthio, 3-chlorophenylthio, 3-bromophenylthio, 4-bromophenylthio, 3,4-dibromophenylthio, 3-chloro-4-fluorophenylthio, 2-fluorophenylthio, 4-hydroxyphenylthio, 3-hydroxyphenylthio, 2,4-dihydroxyphenylthio, 3- or 4-nitrophenylthio, 4-cyanophenylthio, 4-methylphenylthio, 2,4-dimethylphenylthio, 2-methylphenylthio, 4-(iso-propyl)phenylthio, 4-ethylphenylthio, 3-(n-propyl)phenylthio, 2,6-dimethoxyphenylthio, 4-methoxyphenylthio, 3-ethoxyphenylthio, 4-(iso-propoxy)phenylthio, 4-(t-butoxy)phenylthio, 3-ethoxy-4-methoxyphenylthio, a 3- or 4-(trifluoromethyl)phenylthio, 4-carboxyphenylthio, 2,4-di(protected carboxy)phenylthio, 3-(protected hydroxymethyl)phenylthio, 3,4-di(hydroxymethyl)phenylthio, 2-(aminomethyl)-phenylthio, 2,4-di(protected aminomethyl)phenylthio, 3-(methylsulfonylamino)phenylthio, 3-methyl-4-hydroxyphenylthio, 3-chloro-4-hydroxyphenylthio, 2-methoxy-4-bromophenylthio, 4-ethyl-2-hydroxyphenylthio, 3-hydroxy-4-nitrophenylthio, 2-hydroxy-4-chlorophenylthio and the corresponding sulfoxide and sulfone analogs thereof.

A preferred group of examples of the group

$$-\overset{(O)_z}{\overset{\|}{S}}-R_7$$

include: 4-(carboxymethyl)-5-methyl-1,3-thiazol-2-ylthio, 4-(carboxymethyl)-5-methyl-1,3-thiazol-2-ylthio sodium salt, 1,3,4-triazol-5-ylthio, 2-methyl-1,3,4-triazol-5-ylthio, 1H-tetrazol-5-ylthio, 1-methyl-1H-tetrazol-5-ylthio, 1-(1-(dimethylamino)eth-2-ylthio)-1H-tetrazol-5-ylthio, 1-(carboxymethyl)-1H-tetrazol-5-ylthio, 1-(carboxymethyl)-1H-tetrazol-5-ylthio sodium salt, 1-(methylsulfonic acid)-1H-tetrazol-5-ylthio, 1-(methylsulfonic acid)-1H-tetrazol-5-ylthio sodium salt, 1,2,3-triazol-5-ylthio, 1,4,5,6-tetrahydro-5,6-dioxo-4-methyl-as-triazin-3-ylthio, 1,4,5,6-tetrahydro-4-(formylmethyl)-5,6-dioxo-as-triazin-3-ylthio, 2,5-dihydro-5-oxo-6-hydroxy-2-methyl-as-triazin-3-ylthio sodium salt, 2,5-dihydro-5-oxo-6-hydroxy-2-methyl-as-triazin-3-ylthio, tetrazolo[1,5-b]pyridazin-6-ylthio, 8-aminotetrazolo-[1,5-b]pyridazin-6-ylthio, methylthio, phenylthio, phenylsulfonyl, methylsulfonyl, methylsulfoxide, and phenylsulfoxide.

In the above Formula I, $R_2$ can be an acyl group of the formula

$$-COR_8 \quad .$$

Examples of such a group include when $R_8$ is: hydrogen (the formyl group); $C_1$ to $C_6$ alkyl, such as acetyl, sec-butylcarbonyl, t-amylcarbonyl and the like; $C_1$ to $C_6$ substituted alkyl, such as (3-cyanopropyl)carbonyl,

4,5-dichloroamylcarbonyl, 2-carboxy-1-nitroethylcarbonyl and the like; phenyl (the benzoyl group); substituted phenyl, for example, 4-methoxybenzoyl, 2,4-dimethylbenzoyl, 3-nitrobenzoyl, 4-trifluoromethylbenzoyl, 2,4-di(alkyloxycarbonyl)benzoyl, 2-(aminomethyl)benzoyl, 3-hydroxy-4-nitrobenzoyl, and the like; $C_7$ to $C_{12}$ arylalkyl, such phenylmethylcarbonyl, 2-phenylethylcarbonyl, phenyl(t-butyl)carbonyl, 3-phenylamylcarbonyl, and the like; trihalomethyl, such as trifluoroacetyl, trichloroacetyl, tribromoacetyl or triiodoacetyl; $C_7$ to $C_{12}$ substituted arylalkyl, such as 3-(3,4-diiodophenyl)propylcarbonyl, 1-(3-chloro-4-fluorophenyl)ethylcarbonyl, 6-(4-cyanophenyl)hexylcarbonyl, 3-phenyl-1-chloro(sec-butyl)carbonyl, 2-phenyl-2-hydroxyethylcarbonyl, 5-phenyl-2-hydroxyamylcarbonyl, 2-(3-nitrophenyl)-3-ethoxypropylcarbonyl, 5,6-dihydroxy-2-(4-ethyl-2-hydroxyphenyl)hexylcarbonyl, 5-carbamoyl-3-nitro-2-(2,4-dimethoxyphenyl)amylcarbonyl, and the like; perfluoro $C_2$ to $C_4$ alkyl, such as perfluoropropionyl, perfluorobutyryl, perfluoropentanoyl, and the like; amino (the primary amido group); (monosubstituted)amino, such as N-methylamido, N-ethylamido, N-(iso-propyl)amido, N-(n-hexyl)amido, N-phenylamido, N-(4-chlorophenyl)-amido, N-(2-hydroxy-4-bromophenyl)amido, N-benzylamido, N-(2-phenyl(n-propyl))amido, and the like; and (disubstituted)amino, such as N,N-dimethylamido, N,N-methylphenylamido, N,N-(phenyl)(phenethyl)amido, N,N-ethyl-(4-cyanophenyl)amido, N,N-dibenzylamido, N,N-methylethylamido, N,N-methylbenzylamido, and the like.

A preferred group of examples of the acyl group formed with $R_8$ is the acetyl, benzoyl, N-methylamido, N-phenylamido, trifluoroacetyl, trichloroacetyl, tribromoacetyl, and triiodoacetyl groups. An especially preferred group is the acetyl group.

When $R_2$ in the above Formula I is a carboxyl group of the formula

$$-COOR_9$$

examples include groups when $R_9$ is: $C_1$ to $C_6$ alkyl, such as ethoxycarbonyl, sec-butoxycarbonyl, t-amyloxycarbonyl, and the like; $C_1$ to $C_6$ substituted alkyl, such as (3-cyanopropyloxy)carbonyl, 4,5-dichloroamyloxycarbonyl, 2-carboxy-1-nitroethoxycarbonyl, and the like; phenyl (the phenoxycarbonyl group), substituted phenyl, for example, 4-methoxyphenoxycarbonyl, 2,4-dimethylphenoxycarbonyl, 3-nitrophenoxycarbonyl, 4-trifluoromethylphenoxycarbonyl, 2,4-di(methoxycarbonyl)phenoxycarbonyl, 2-(aminomethyl)phenoxycarbonyl, 3-hydroxy-4-nitrophenoxycarbonyl, and the like; $C_7$ to $C_{12}$ arylalkyl, such benzyloxycarbonyl, 2-phenylethoxycarbonyl, phenyl(t-butoxy)carbonyl, 3-phenylamyloxycarbonyl, and the like; trihalomethyl, such as trifluoromethoxycarbonyl, trichloromethoxycarbonyl, tribromomethoxycarbonyl or triiodomethoxycarbonyl; or $C_7$ to $C_{12}$ substituted arylalkyl, such as 3-(3,4-diiodophenyl)propoxycarbonyl, 1-(3-chloro-4-fluorophenyl)ethoxycarbonyl, 6-(4-cyanophenyl)hexyloxycarbonyl, 3-phenyl-1-chloro(sec-butoxy)carbonyl, 2-phenyl-2-hydroxyethoxycarbonyl, 5-phenyl-2-hydroxyamyloxycarbonyl, 2-(3-nitrophenyl)-3-ethoxypropoxycarbonyl, 5,6-dihydroxy-2-(4-ethyl-2-hydroxyphenyl)hexyloxycarbonyl, and 5-carbamoyl-3-nitro-4-(2,4-dimethoxyphenyl)amyloxycarbonyl and the like.

Further examples of the above $-COOR_9$ group are when $R_9$ is: an organic or inorganic cation, such ammonium carboxylate, procaine carboxylate, (phenylethylbenzylammonium)carboxylate, phenylglycine carboxylate, lysine carboxylate, lithium carboxylate, potassium carboxylate, sodium carboxylate and the like; a carboxy protecting group, such as allyl carboxylate, p-methoxybenzyl carboxylate, di-(4-methoxy)benzhydryl carboxylate, benzhydryl carboxylate, 2,2,2-trichloroethyl carboxylate, trimethylsilyl carboxylate, (t-butyl)dimethylsilyl carboxylate, β-(trimethylsilyl)ethyl carboxylate, trityl carboxylate, 4,4',4''-trimethoxytrityl carboxylate, p-toluenesulfonylethyl carboxylate, and the like; a non-toxic, metabolically-labile ester-forming group, such as methoxymethyl carboxylate, 5-methyl-2-oxo-1,3-dioxolen-4-ylmethyl carboxylate, ethylthiomethyl carboxylate, pivaloyloxymethyl carboxylate, 3-phthalidyl carboxylate, 1-(ethoxycarbonyloxy)ethyl carboxylate, 1-(methylaminocarbonyloxy)ethyl carboxylate, and the like.

A preferred group of examples of the carboxy group $-COOR_9$ is when $R_9$ is a $C_1$ to $C_6$ alkyl group, a carboxy protecting group, hydrogen or an organic or inorganic cation. An especially preferred group of examples of the above carboxy group is when $R_9$ is methyl, ethyl, hydrogen, allyl, t-butyl, 4-nitrobenzyl, or sodium.

Examples of the group $-COOR_{10}$ are given above in conjunction with the carboxy group $-COOR_9$.

A preferred group of example of the group $-COOR_{10}$ occurs when $R_{10}$ is a $C_1$ to $C_6$ alkyl group. An especially preferred carboxyl group of the above formula is ethyl carboxylate.

Examples of the group

$-SO_2Y$

include $C_1$ to $C_6$ alkylsulfonyl groups such as methylsulfonyl, ethylsulfonyl, (t-amyl)sulfonyl and (n-hexyl)-sulfonyl, $C_7$ to $C_{12}$ arylalkyl sulfonyl groups such as (2-phenyl)propylsulfonyl, benzylsulfonyl, 1-phenyl(n-amyl)sulfonyl and 4-phenyl(n-butyl)sulfonyl; $C_1$ to $C_6$ substituted alkylsulfonyl groups such as cyanomethylsulfonyl, 2-hydroxyethylsulfonyl, 2-nitropropylsulfonyl, 2-carbamoyl(sec-butyl)sulfonyl, 5-chloroamylsulfonyl, 4-carboxyamylsulfonyl, 6-carbamoyloxyhexylsulfonyl, 2-methoxyethylsulfonyl, iso-propoxy(t-butyl)sulfonyl, 2-aminoethylsulfonyl, 2,5-dihydroxyamylsulfonyl, 3,3-dibromo(n-butyl)sulfonyl, 3-chloro-2-iodopropylsulfonyl, and 4-acetoxy-6-fluorohexylsulfonyl; $C_7$ to $C_{12}$ substituted arylalkylsulfonyl groups such as 3-(3,4-diiodophenyl)propylsulfonyl, 1-(3-chloro-4-fluorophenyl)ethylsulfonyl, 6-(4-cyanophenyl)hexylsulfonyl, 3-phenyl-1-chloro(sec-butyl)sulfonyl, 2-phenyl-2-hydroxyethylsulfonyl, 5-phenyl-2-hydroxyamylsulfonyl, 2-(3-nitrophenyl)-3-ethoxypropylsulfonyl, 5,6-dihydroxy-2-(4-ethyl-2-hydroxyphenyl)hexylsulfonyl and 5-carbamoyl-3-nitro-4-(2,4-dimethoxyphenyl)amylsulfonyl; phenylsulfonyl, and (substituted phenyl)sulfonyl groups. Examples of the latter (substituted phenyl)sulfonyl groups include a mono- or di(substituted)phenyl group such as 4-chlorophenylsulfonyl, 2,6-dichlorophenylsulfonyl, 2,5-dichlorophenylsulfonyl, 3,4-dichlorophenylsulfonyl, 3-chlorophenylsulfonyl, 3-bromophenylsulfonyl, 4-bromophenylsulfonyl, 3,4-dibromophenylsulfonyl, 3-chloro-4-fluorophenylsulfonyl, 2-fluorophenylsulfonyl, 4-hydroxyphenylsulfonyl, 3-hydroxyphenylsulfonyl, 2,4-dihydroxyphenylsulfonyl, 3- or 4-nitrophenylsulfonyl, 4-cyanophenylsulfonyl, 4-methylphenylsulfonyl, 2,4-dimethylphenylsulfonyl, 2-methylphenylsulfonyl, 4-(iso-propyl)phenylsulfonyl, 4-ethylphenylsulfonyl, 3-(n-propyl)phenylsulfonyl, 2,6-dimethoxyphenylsulfonyl, 4-methoxyphenylsulfonyl, 3-ethoxyphenylsulfonyl, 4-(iso-propoxy)phenylsulfonyl, 4-(t-butoxy)phenylsulfonyl, 3-ethoxy-4-methoxyphenylsulfonyl, 3- or 4-(trifluoromethyl)phenylsulfonyl, 4-carboxyphenylsulfonyl, 2,4-di(protected carboxy)phenylsulfonyl, 3-(protected hydroxymethyl)phenylsulfonyl, 3,4-di(hydroxymethyl)phenylsulfonyl, 2-(aminomethyl)phenylsulfonyl, 2,4-(protected aminomethyl)phenylsulfonyl, 3-(methylsulfonylamino)phenylsulfonyl, 3-methyl-4-hydroxyphenylsulfonyl, 3-chloro-4-hydroxyphenylsulfonyl, 2-methoxy-4-bromophenylsulfonyl, 4-ethyl-2-hydroxyphenylsulfonyl, 3-hydroxy-4-nitrophenylsulfonyl, 2-hydroxy-4-chlorophenylsulfonyl and the like.

A preferred group of examples of the group

$$-SO_2Y$$

is the methylsulfonyl, phenylsulfonyl, 4-methylphenylsulfonyl and benzylsulfonyl groups, with the 4-methylphenylsulfonyl and the phenylsulfonyl groups being the more preferred groups.

Examples of the compounds of Formula I are listed below in Table I:

Table I

Compounds of the Formulas

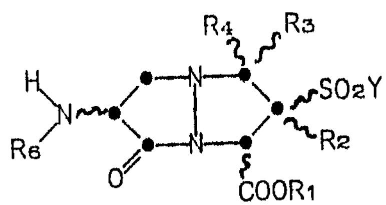

and

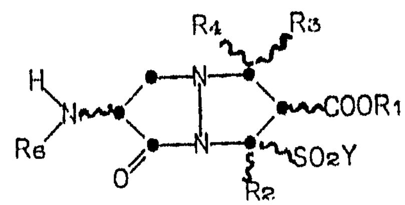

| $R_1$ | $R_2$ | $R_3$ | $R_4$ | $R_6$ | Y |
|---|---|---|---|---|---|
| allyl | $CH_2Cl$ | H | H | t-BOC[1] | 4-methylphenyl |
| allyl | $CH_2OTHP$[2] | H | H | " | " |
| allyl | $CH_2OAc$ | H | H | " | " |
| pNB[3] | phenyl | methyl | H | AOC[4] | methyl |
| pMB[5] | 3-(trifluoromethyl)-phenyl | H | methyl | benzyloxycarbonyl | 2-chloroethyl |
| benzhydryl | ethyl | phenyl | H | formyl | benzyl |
| t-butyl | phenylsulfonyl | 2-trifluoro-methylphenyl | H | chloroacetyl | 4-chlorobenzyl |

Table I cont'd.

| $R_1$ | $R_2$ | $R_3$ | $R_4$ | $R_6$ | Y |
|---|---|---|---|---|---|
| 2,2,2-trichloro-ethyl | n-propyl-sulfoxide | ethoxycarbonyl | H | 2-(trimethylsilyl)-ethoxycarbonyl | phenyl |
| trimethylsilyl | trifluoromethyl | benzyl | H | trimethylsilyl | 4-methoxyphenyl |
| cinnamyl | 1-methyltetrazol-5-ylthio | H | H | trityl | n-propyl |
| acetoxymethyl | formyl | H | phenyl | H | benzyl |
| trityl | acetyl | H | benzyl | cyclopentanyloxy-carbonyl | 3-phenylpropyl |
| allyl | acetyl | H | H | t-Boc | phenyl |
| allyl | acetyl | H | H | H | phenyl |
| β-(trimethyl-silyl)ethyl | perfluorobutyryl | 3-nitrobenzyl | H | 5-benzisoxalyl-methoxycarbonyl | 3-phenylbutyl |
| t-amyl | trichloroacetyl | 2,5-dimethyl-phenyl | H | 2,2,2-trichloro-ethoxycarbonyl | benzyl |
| 2,4,6-trimethyl-benzyl | benzoyl | H | 4-methyl-phenyl | 4-methoxybenzyloxy-carbonyl | methyl |

Table I cont'd.

| $R_1$ | $R_2$ | $R_3$ | $R_4$ | $R_6$ | Y |
|---|---|---|---|---|---|
| 2-phenylprop-2-yl | 4-nitrobenzoyl | 2,3-dibromopropyl | H | 4-cyanobenzyloxy-carbonyl | 3-cyanopropyl |
| t-butyldimethyl silyl | 2-phenylacetyl | H | methyl | diphenyl-phosphine oxide | 3-ethylphenyl |
| pentamethyl-benzyl | 2-(3-chlorophenyl)-acetyl | H | H | isobornyloxy-carbonyl | 3-phenyl-2-chloro-propyl |
| phenacyl | methoxycarbonyl | AOC | H | 1,1-diphenyleth-1-yloxycarbonyl | ethyl |
| p-nitrobenzyl-sulfonylethyl | 2-fluoroethyloxy carbonyl | iso-butyl | H | trichloroacetyl | 4-phenylbutyl |
| benzyl | benzyloxycarbonyl | n-propyl | H | bromoacetyl | hexyl |
| 3,4-dimethoxy-benzyl | 4-methylbenzyloxy-carbonyl | H | n-butyl | 4-nitrobenzyloxy-carbonyl | 1-fluorethyl |
| benzhydryl | phenoxycarbonyl | 3-phenyl-2-chloropropyl | H | 2-(methylsulfonyl)-ethyloxycarbonyl | 2-nitropropyl |
| 1-(trimethyl-silyl)methyl-prop-1-en-3-yl | 3-nitro-phenoxycarbonyl | H | H | 2,4-dichlorobenzyl-oxycarbonyl | phenyl |

Table I cont'd.

| $R_1$ | $R_2$ | $R_3$ | $R_4$ | $R_6$ | Y |
|---|---|---|---|---|---|
| pivaloyloxy-methyl | sodium carboxylate | H | H | H | phenyl |
| ethoxyethyl | ethoxyethyl carboxylate | H | H | H | 4-methylphenyl |
| methylthio-methyl | 1,3,4-thiadiazol-2-ylthiomethyl | H | H | AOC | benzyl |
| t-butyl | N-phenylamido | H | H | t-Boc | phenyl |
| allyl | N-phenylamido | H | H | t-Boc | phenyl |
| t-butyl | N-methylamido | H | H | t-Boc | phenyl |
| allyl | N-methylamido | H | H | t-Boc | phenyl |

[1] t-BOC = t-butoxycarbonyl

[2] THP = tetrahydropyranyl

[3] pNB = p-nitrobenzyl

[4] AOC = allyloxycarbonyl

[5] pMB = p-methoxybenzyl

One of two preferred groups of the above examples is when either $R_5$ or $R_6$ is hydrogen and the other is an amino protecting group. The amino-protected group contains a preferred group of compounds wherein $R_2$ is (a) hydrogen, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano or (b) a group of the formula

$$-\overset{(O)_z}{\overset{\|}{S}}-R_7 \quad ,$$

and especially so when Y is phenyl or substituted phenyl and $R_3$ and $R_4$ are both hydrogen. A more preferred group of compounds occurs when Z is a group of the formula

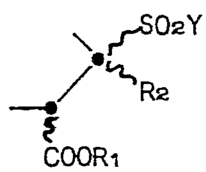

and especially so when $R_1$ is a carboxy-protecting group.

One series of compounds worthy of note in the above more preferred group occurs when $R_2$ is hydrogen and furthermore when either $R_5$ or $R_6$ is hydrogen and the other is t-butoxycarbonyl, and Y is 4-methylphenyl. Another series of compounds of note occurs when $R_2$ is methylthio, and especially so when $R_1$ is t-butyl, $R_5$ or $R_6$ is hydrogen and the other is t-butoxycarbonyl, and Y is 4-methylphenyl.

The second of the two preferred groups of compounds of the compounds of Table 1 are the 7-(S)-compounds, in other words, compounds of Formula 1 represented by the formula:

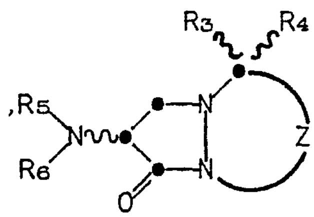

A preferred group of the 7-(S) compounds is when either $R_5$ or $R_6$ is hydrogen and the other is an amino-protecting group. The group of amino-protected compounds contains a preferred group of compounds wherein $R_2$ is a group of the formula

$$-COR_8,$$

and especially so when Y is phenyl or substituted phenyl and $R_3$ and $R_4$ are each hydrogen. A more preferred group of compounds occurs when Z is a group of the formula:

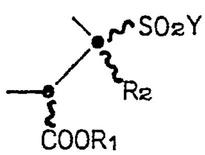

wherein $R_1$ is a carboxy-protecting group.

Especially desirable 7-(S) compounds in the group of above preferred compounds occurs when $R_7$ is an acetyl group, i.e., when $R_8$ is methyl, and especially so when $R_1$ is an allyl group, either $R_5$ or $R_6$ is hydrogen and the other is t-butoxycarbonyl, and Y is a phenyl group.

The 7-substituted-2,3-(dihydro)-bicyclic pyrazolidinones of Formula I can be synthesized with a 1,3-dipolar cycloaddition reaction. The cycloaddition reaction is represented by Scheme 1:

Scheme 1

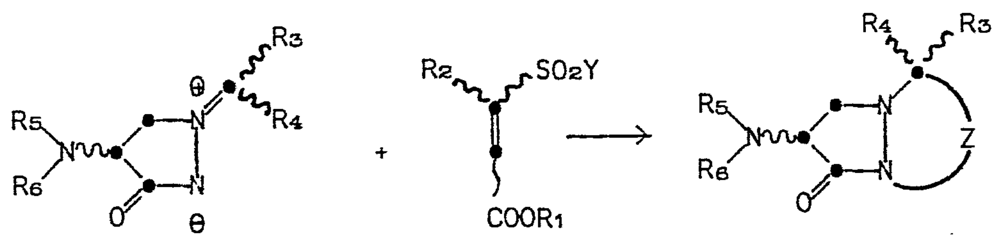

ylide ethylene I

In Scheme 1, $R_1$, $R_2$, $R_3$, $R_4$, $R_5$, $R_6$, Z and Y are as defined for Formula I, except that $R_5$ and $R_6$ of the ylide are not both hydrogen. In conjunction with the above process, it is preferable to derivatize with protecting groups any acidic groups on the ylide, ethylene, or bicyclic pyrazolidinone ring system of Formula I represented by $R_1$, $R_2$, $R_3$, $R_4$ or Y. One such acidic group is the carboxyl group.

The reaction in the above Scheme 1 is carried out in aprotic, preferably polar solvents. Examples of such solvents are the chlorinated hydrocarbons, the aromatic hydrocarbons and alkyl or aromatic cyano solvents. A preferred solvent for the above reaction is 1,2-dichloroethane.

The temperature for the reaction is not critical. It is preferred that the reaction be carried out between about room temperature to about the reflux temperature of the solvent. A more preferred temperature is approximately the reflux temperature of the solvent.

The reaction usually requires a period of about 1 to about 168 hours. The optimal reaction time can be determined by monitoring the progress of the reaction by conventional means such as chromatographic techniques (thin layer chromatography, high performance liquid chromatography, or column chromatography), spectroscopic methods (such as infrared spectroscopy, nuclear magnetic resonance spectrometry and mass spectrometry), or a combination of the two methods.

The usual stoichiometry for the reaction in the above Scheme 1 is a 1:1 ratio of ylide to ethylene reactant. Of course, an excess of either reactant is permissible. The order of addition of either reactant is not critical.

The regiospecificity of the above cycloaddition reaction is such that the 3-(sulfonyl) regioisomer is the predominant product. Selected examples have shown that the (E)-vinyl sulfone isomer of the formula

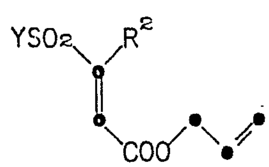
,

preferably Y is phenyl and $R^2$ is acetyl, enhances the yield of the 3-(sulfonyl) adduct.

The stereospecificity of the reaction at $C_2$ and $C_3$ of the bicyclic pyrazolidinone is unpredictable. The reaction usually produces a mixture of stereoisomers at these positions. The stereospecificity of the cycloaddition at $C_7$ of adduct (Formula I) is determined by the stereochemistry of the $C_4$ position of the ylide. Thus, if a 4-(S)-ylide is the starting material, the cycloadduct (Formula I) has 7-(S) stereochemistry.

The compounds of Formula I can be converted to 7-substituted bicyclic pyrazolidinone antimicrobials (and the corresponding intermediates) according to the elimination reaction set forth below in Scheme 2:

Scheme 2

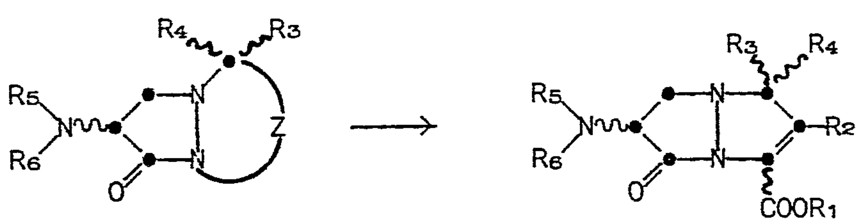

I unsaturated system

In the above Scheme 2, $R_1$, $R_2$, $R_3$, $R_4$, $R_5$, $R_6$, Z, and Y are as defined for Scheme 1. Again, it is preferred that acidic groups at $R_1$, $R_2$, $R_3$, $R_4$ and Y be derivatized with a protecting group. An example of such an acidic group is a carboxylic acid.

For the above elimination reaction, the preferred solvent is dichloromethane. The elimination is conducted at a temperature from about -78°C to about

room temperature. A non-nucleophilic base, such as 1,8-diazabicyclo[5.4.0]undec-7-ene ("DBU"), 1,5-diazabicyclo[4.3.0]non-5-ene ("DBN"), triethylamine, or N-methylmorpholine, is used to eliminate the elements of (alkyl or aryl)sulfinic acid. An excess of the non-nucleophilic base in relation to the "saturated system" is normally used. The preferred base is N-methylmorpholine, as it does not racemize the C-7 position of either the saturated cycloadduct (Formula I) or the unsaturated system of Scheme 2.

The compounds produced by the reaction in Scheme 2 ("unsaturated system") are intermediates to antimicrobial compounds. In order to convert the intermediates to the corresponding antimicrobial compounds, it is necessary to replace the amino-protecting group at either $R_5$ or $R_6$ with an acyl group derived from a $C_1$ to $C_{30}$ carboxylic acid. The acyl groups employed are typically those used to achieve the same purpose when bonded to the 6-amino group of a penicillin or a 7-amino group of a cephalosporin.

The first step for the acylation of a 7-(protected amino) bicyclic pyrazolidinone compound ("7-protected amino nucleus") products of Scheme 2 is the removal of the amino-protecting group. The conditions for the removal of these groups are well known in the cephalosporin and penicillin arts. For example, the trimethylsilyl protecting group is removed by simple hydrolysis, the t-butoxycarbonyl group is removed by acidic hydrolysis or acidolysis (trifluoroacetic acid or a mixture of hydrochloric acid in acetic acid respectively) and the allyloxycarbonyl group is removed as a palladium complex.

Removal of the acid-labile amino protecting groups usually yields the 7-amino nucleus as a salt. The salt of the nucleus is neutralized by conventional procedures before acylation. For instance, the removal of the t-butoxycarbonyl group with trifluoroacetic acid leaves the trifluoroacetate salt of the resultant 7-amino nucleus. The salt is taken up in tetrahydrofuran and bis(trimethylsilyl)trifluoroacetamide is added to yield the corresponding neutralized 7-compound. The neutralized compound can be isolated or acylated *in situ*. The removal of the t-butoxycarbonyl group with a mixture of hydrochloric acid in acetic acid yields the hydrochloride salt of the nucleus. The hydrochloride salt may be taken up in water and acetonitrile and a solution of $K_2HPO_4$ added to give the amino nucleus.

The methods for the acylation of the neutralized 7-amino nucleus with the acyl side chain are similar to the methods for the acylation of 6-aminopenicillanic acid, 7-aminodesacetoxycephalosporanic acid and 7-aminocephalosporanic acid. One method is to simply combine the 7-amino nucleus with an acid chloride or acid bromide. The acid chloride or acid bromide may be formed *in situ*. Another method is to combine the 7-amino nucleus with the free carboxylic acid form of the side chain (or its acid salt) and a condensing agent. Suitable condensing agents include N,N'-disubstituted carbodiimides such as N,N'-dicyclohexylcarbodiimide, N,N'-diethylcarbodiimide, N,N'-di-(n-propyl)-carbodiimide, N,N'-di-(iso-propyl)carbodiimide, N,N'-diallylcarbodiimide, N,N'-bis(p-dimethylaminophenyl)-carbodiimide, N-ethyl-N'-(4''-ethylmorpholinyl)carbodiimide and the like. Other suitable carbodiimides are disclosed by Sheehan in U.S. Patent No. 2,938,892 and by Hofmann et. al. in U.S. Patent No. 3,065,224. Azolides, such as N,N'-carbonyldiimidazole and N,N'-thionyldiimidazole, may also be used as condensing agents. Dehydrating agents such as phosphorus oxychloride, the alkoxyacetylenes and 2-halogenopyridinium salts (such as 2-chloropyridinium methyl iodide, 2-fluoropyridinium methyl iodide, and the like) may be used to couple the free acid or its acid salt with the 7-amino nucleus.

Another acylation method entails first converting the free carboxylic acid form (or the corresponding salt) of the acyl side chain to the active ester derivative which is in turn used to acylate the nucleus. The active ester derivative is formed by esterifying the free acid form with groups such as p-nitrophenol, 2,4-dinitrophenol, trichlorophenol, pentachlorophenol, N-chlorosuccinimide, N-chloro maleic imide, N-chlorophthalimide, 2-chloro-4,6-dimethoxytriazine, 1-hydroxy-1H-benzotriazole or 1-hydroxy-6-chloro-1H-benzotriazole. The active ester derivatives can also be mixed anhydrides, formed with groups such as methoxycarbonyl, ethoxycarbonyl, iso-butoxycarbonyl, trichloromethylcarbonyl or iso-but-2-ylcarbonyl and the carboxylic acid form of the acyl side chain. The mixed anhydrides are formed by acylating the carboxylic acid function of the acyl side chain.

Alternatively, the 7-amino nucleus can be acylated with the N-ethoxycarbonyl-2-ethoxy-1,2-dihydroquinoline (EEDQ) derivative of the acyl side chain. In

general, the free acid form of the acyl side chain and EEDQ are reacted in an inert, polar organic solvent (e.g. tetrahydrofuran, acetonitrile, etc.). The resultant EEDQ derivative is used in situ to acylate the 7-amino nucleus.

Once the 2,3-unsaturated bicyclic pyrazolidinones are acylated with the appropriate acyl group derived from a $C_1$ to $C_{30}$ carboxylic acid, they are converted to the corresponding antimicrobial final product form by removing any remaining amino, hydroxy and/or carboxy-protecting groups on the molecule. As discussed above, such removal methods are well known in the cephalosporin, penicillin and peptide arts. Once the carboxy groups are deprotected, the non-toxic, metabolically-labile ester-forming group ("oral ester") may be put in place on the desired carboxy groups at $R_1$, $R_2$, and $R_3$ or $R_4$. The methods for making the oral ester derivatives are well known in the cephalosporin and penicillin art.

The pyrazolidinium ylide starting materials for the above cycloaddition reaction in Scheme 1 are synthesized according to the process depicted below in Scheme 3. The process yields a mixture of $C_4$ enantiomers of the ylide.

Scheme 3

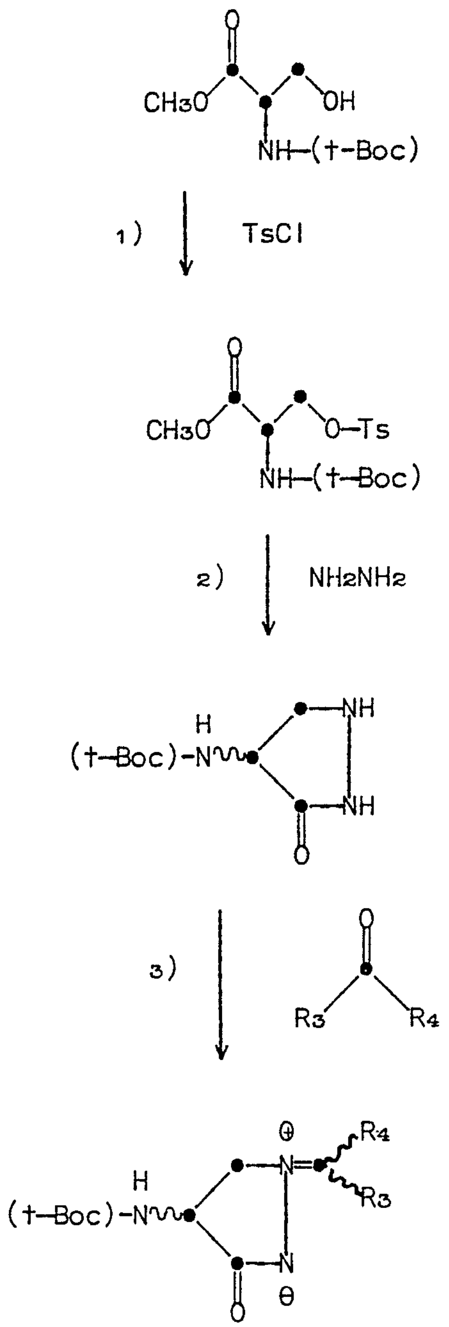


protected serine

tosyl serine

diazolidine

ylide

The above Scheme depicts the synthesis of an enantiomeric mixture 4-(t-butoxycarbonylamino) ylide starting materials. Ylide starting materials with different amino-protecting groups are obtained from serine derivatized with a carboxy-protecting group other than t-butoxycarbonyl.

The first step in the synthesis of the enantiomeric mixture of ylide starting materials, represented by Reaction 1 in the above Scheme, is the tosylation of the hydroxy group of the protected serine derivative. The serine derivative is tosylated in methylene chloride with p-toluenesulfonyl chloride in the presence of a catalytic amount of 4-dimethylaminopyridine and greater than one equivalent of pyridine. The reaction mixture is stirred at room temperature overnight.

The tosylated serine obtained is cyclized to give the diazolidine. The cyclization, represented by Reaction 2, entails adding the tosyl serine to a solution of 97% hydrazine in methylene chloride under nitrogen. The mixture is stirred at room temperature for approximately five hours.

The final reaction in the synthesis of the enantiomeric mixture of pyrazolidinium ylide starting materials comprises the condensation of an aldehyde with a diazolidine. When $R_3$ and $R_4$ are different those skilled in the art will recognize that this final reaction will produce a mixture of E and Z isomers. The unsubstituted ylide (when $R_3$ and $R_4$ are hydrogen) is synthesized by combining the diazolidene reagent and 37% aqueous formaldehyde in methanol and stirring the mixture for twenty minutes at room temperature.

The stereospecific synthesis of the pyrazolidinium ylides of Formula I is diagramed below in Scheme 4.

Scheme 4

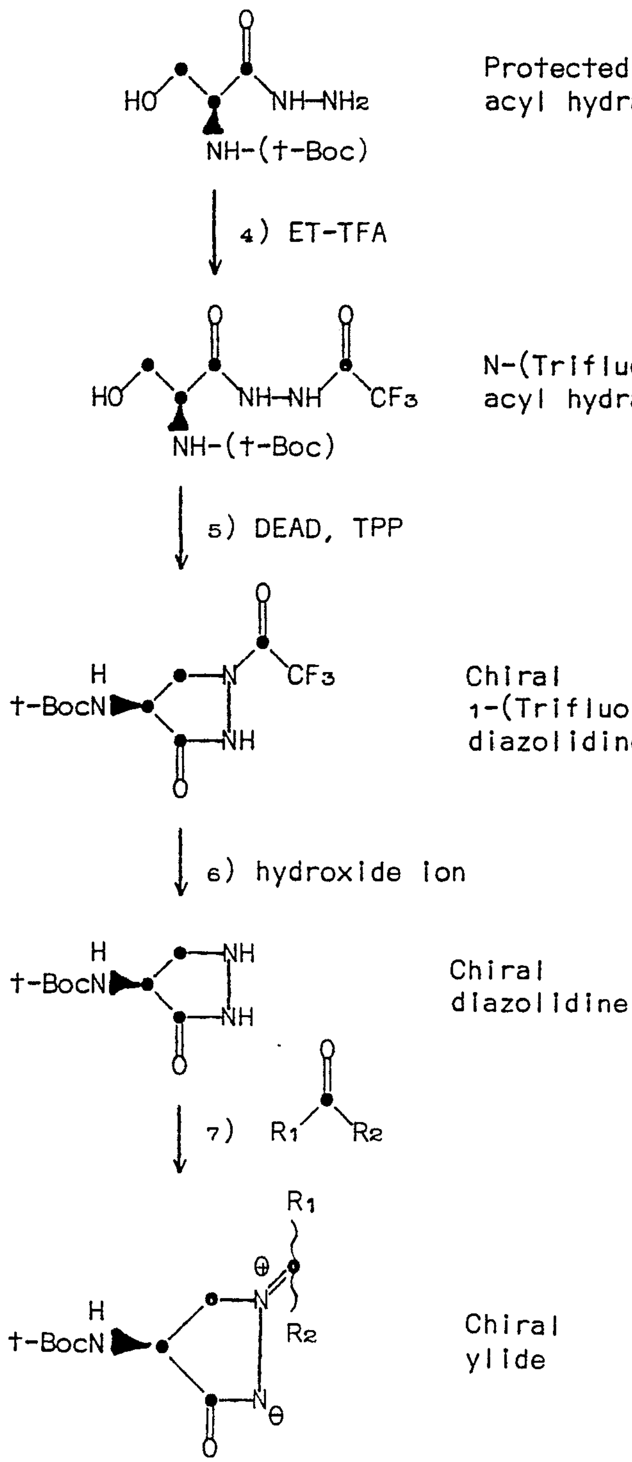


Protected serine acyl hydrazide

N-(Trifluoroacetyl) acyl hydrazide

Chiral 1-(Trifluoroacetyl) diazolidine

Chiral diazolidine

Chiral ylide

The above Scheme depicts the synthesis of chiral 4-(S)-(t-butoxycarbonylamino) ylide compounds. Ylide compounds with the 4-(R) configuration are synthesized by starting with the protected D-serine acyl hydrazide instead of the L-isomer depicted above. Both 4-(R) or 4-(S) compounds with amino-protecting groups other than t-butoxycarbonyl are synthesized from the corresponding serine enantiomer substituted with an amino-protecting group other than t-butoxycarbonyl.

The protected serine acyl hydrazide precursor of Scheme 4 is synthesized in a procedure analogous to B. Iselin and R. Schwyzer, Helv. Chim. Acta, **44**, p. 169 (1961). The precursor is then acylated with the trifluoroacetyl moiety, as set forth in Reaction 4 in the Scheme. The hydrazide precursor is acylated with an excess of ethylthio trifluorothioacetate ("ET-TFA") in ethanol. The reaction mixture is stirred at room temperature for 65 hours.

The N-(trifluoroacetyl) acyl hydrazide obtained from Reaction 4 is cyclized with triphenylphosphine ("TPP") and diethyl azodicarboxylate ("DEAD"), as depicted above in Reaction 5.

The stoichiometry of the cyclization of Reaction 5 has the N-(trifluoroacetyl) acyl hydrazide, phosphine and diethyl azodicarboxylate reagent present in at least approximately a 1:1:1 molar ratio. The reaction will proceed in the presence of molar excesses above this ratio of any of the reactants.

The cyclization is initiated by first combining (in any order) the solvent, the N-(trifluoroacetyl) acyl hydrazide and the phosphine, and secondly adding the azodicarboxylate reagent.

The temperature of Reaction 5 is not a critical parameter. The cyclization can be carried out at a temperature from approximately the freezing point to approximately the reflux temperature of the solvent. The preferred temperature is approximately room temperature.

The duration of Reaction 5 can be from approximately five minutes to approximately twenty four hours. The progress of the cyclization can be monitored by standard methods (e.g., thin layer chromatography, high performance liquid chromatography, etc.) The process is stopped when the monitoring method demonstrates that the reaction is substantially complete.

The solvents for the cyclization are aromatic hydrocarbon solvents such as benzene, toluene or xylenes; ethers such as diethyl ether, tetrahydrofuran, or 1,4-dioxane; chlorinated hydrocarbons such as methylene chloride, chloroform, carbon tetrachloride, dichloroethane, or chlorobenzene; amides such as dimethylformamide and dimethylacetamide; and other solvents such as hexamethylphosphoramide. Tetrahydrofuran is the preferred solvent. It is also desirable, but not essential, to dry and deoxygenate the solvent before use in the process.

While Reaction 5 in the above Scheme depicts the use of diethyl azodicarbonylate, the dimethyl and di(iso-propyl)azodicarboxylate analogs can also be used in the reaction.

The chiral 1-(trifluoroacetyl)diazolidine obtained from Reaction 5 is deacylated with dilute sodium hydroxide solution. The deacylation is represented as Reaction 6 in the Scheme. The deacylation entails generally suspending the chiral 1-(trifluoroacetyl)diazolidine in water and adding at least two equivalents of a dilute aqueous solution of either sodium hydroxide or potassium hydroxide. For instance, a two-fold excess of 1M sodium hydroxide solution can be used. It is preferred to have the initial pH of the solution from between about 11 to about 12. The resultant solution can be stirred from about 10 minutes to about 3 hours at a temperature from about 10°C to about 25°C. When the reaction is substantially complete the reaction solution is neutralized by the addition of dilute acid, such as 1N hydrochloric acid.

The optimal reaction time for the deacylation can be determined by monitoring the progress of the reaction with conventional chromatographic techniques (thin layer chromatography, high performance liquid chromatography, or column chromatography), or spectroscopic methods, (such as infrared spectroscopy, nuclear magnetic resonance spectrometry and mass spectrometry) or a combination of both methods. A preferred time period is from between about 30 minutes to about 1.5 hours.

The final reaction of Scheme 4, wherein the chiral diazolidines are converted to the chiral pyrazolidinium ylides, is carried out using the conditions described for the analogous reaction (Reaction 3) in Scheme 3.

The synthesis of the above diazolidine and pyrazolidinium ylide starting materials are further described by L.N. Jungheim and R. E. Holmes, European

Patent Applications Nos. __________ and ___________ (references X-6711A and X-6739A) respectively, filed this even date, herein incorporated by reference, which applications are related to U.S. Patent Application Nos. 728,734 and 728,733 respectively filed April 30, 1985. Those skilled will appreciate that amino protecting groups other than t-BOC could be used in the above sequence.

The ethylene starting materials in Scheme 1 are made by methods known in the art and/or commercially available. The synthesis of some ethylene starting materials are also described in the Experimental Section below.

The 2,3-unsaturated bicyclic pyrazolidinone antimicrobial compounds in Scheme 2 ("unsaturated system") inhibit the growth of certain organisms pathogenic to man and animals. The antimicrobial compounds of the unsaturated system are compounds wherein the various amino, hydroxy and/or carboxy protecting groups have been removed. The antimicrobial activity can be demonstrated *in vitro* using standard tube-dilution techniques. The *in vitro* tests demonstrate that, in general, the 7-(S) isomers have better antimicrobial activity than the corresponding 7-(R) isomers or a mixture of the two isomers. Representative pathogens which are sensitive to the antimicrobial compounds include *Staphylococcus aureus* X1.1, *Streptococcus pyogenes* C203, *Streptococcus pneumoniae* Park, *Hemophilus influenzae* 76 (ampicillin resistant), *Escherichia coli* N10, *Escherichia coli* EC14, *Escherichia coli* TEM (b-lactamase producer), *Klebsiella*

pneumoniae X26, Klebsiella pneumoniae KAE (β-lactamase producer), Klebsiella pneumoniae X68, Enterobacter aerogenes C32, Enterobacter aerogenes EB17, Enterobacter cloacae EB5 (non-β-lactamase producer), Salmonella typhi X514, Salmonella typhi B35, Serratia marcescens X99, Serratia marcescens SE3, Proteus morganii PR15, Proteus inconstans PR33, Proteus rettgeri C24, Citrobacter freundii CF17, and the like.

The 2,3-unsaturated bicyclic pyrazolidinone antimicrobial compounds are useful for the therapeutic or prophylactic treatment of infections in warm-blooded animals caused by gram-positive, gram-negative and acid-fast bacteria.

The antimicrobial compounds can be administered orally, parenterally (e.g. intravenously, intramuscularly or subcutaneously) or as a topical ointment or solution in treating bacterial infections of warm-blooded animals.

Further description of the synthesis and properties of the 7-substituted 2,3-unsaturated bicyclic pyrazolidinone antimicrobials and the corresponding intermediates depicted in Scheme 2 are found in L. N. Jungheim, S. K. Sigmund, C. J. Barnett, R. E. Holmes and R. J. Ternansky, European Patent Application No. __________ (reference X-5841A) filed this even date, herein incorporated by reference, which in turn is related to U.S. Patent Application No. 729,021, filed April 30, 1985.

In the following Preparations and Examples, the terms melting point, nuclear magnetic resonance spectra, field desorption mass spectra, electron impact mass spectra, infrared spectra, ultraviolet spectra, elemental analysis, and thin layer chromatography are abbreviated m.p., n.m.r., f.d.m.s., m.s., i.r., u.v., anal. and TLC, respectively. In addition, the adsorption maxima listed for the i.r. spectra are only those of interest and not all of the maxima observed.

The abbreviations THF, DMF, TFA, BSTFA and DBU stand for tetrahydrofuran, dimethylformamide, trifluoroacetate, N,O-bis(trimethylsilyl)trifluoroacetamide and 1,8-diazabicyclo[5.4.0]undec-7-ene, respectively.

In conjunction with the n.m.r. spectra, the following abbreviations are used: "s" is singlet, "d" is doublet, "dd" is doublet of doublets, "t" is triplet, "q" is quartet, "m" is multiplet, "dm" is a doublet or multiplets, and "br. s", "br. d" and "br. t" are broad singlet, doublet and triplet, respectively. "J" indicates the coupling constant in Hertz. "DMSO/$d_6$" is dimethyl sulfoxide where all protons have been replaced with deuterium.

The n.m.r. spectra were obtained either on a Varian Associates EM-390 90 MHz instrument, on a Jeol FX-90Q 90 MHz instrument, on a Brüker Corp. 270 MHz instrument or on a General Electric QE-300 MHz instrument. The chemical shifts are expressed in δ values (parts per million downfield from tetramethylsilane). The field desorption mass spectra were taken on a Varian-MAT 731 Spectrometer using carbon dendrite

emitters. Election impact mass spectra were obtained on a CEC 21-110 instrument from Consolidated Electrodynamics Corporation. Infrared spectra were obtained on a Perkin-Elmer 281 instrument. Ultraviolet Spectra were obtained on a Cary 118 instrument. Specific rotations were obtained on a Perkin-Elmer Q-41 instrument. Thin layer chromatography was carried out on E. Merck silica gel plates. Melting points are uncorrected.

## Experimental Section

### Preparation 1

Methyl 3-(p-Toluenesulfonate)-2-(S)-(t-Butoxycarbonylamino)Propionate

Methyl (3-hydroxy)-2-(S)-(t-butoxycarbonylamino)propionate (58 g, 196 mmol), dry methylene chloride (150 ml), p-toluenesulfonyl chloride (43.35 g, 227.4 mmol), 4-(dimethylamino)pyridine (2.4 g, 19.6 mmol) and pyridine (30 ml, 371 mmol) were combined and stirred at room temperature overnight. The reaction solution was concentrated *in vacuo* to a pale yellow oil. The oil was stored *in vacuo* overnight, then the white solid that formed was isolated to give 75.33 g of crude product. The product was triturated in petroleum ether (approximately 200 ml) to yield methyl 3-(p-toluenesulfonate)-2-(S)-(t-butoxycarbonylamino)propionate: nmr: ($CDCl_3$, 90 MHz): δ 7.72, 7.31 (2x dd, 4, aromatic protons), 5.26 (m, 1, nitrogen proton), 4.48 (m, 1, C-2 proton),

4.32 (m, 2, C-3 protons), 3.68 (s, 3, methyl protons of methyl ester), 2.44 (s, 3, methyl protons of toluene moiety), 1.40 (s, 9, t-butyl moiety); i.r. ($CHCl_3$): 3435, 3019, 1753, 1711, 1502, 1369, 1351, 1250, 1215, 1190, 1177 $cm^{-1}$; m.s.: $M^+$= 279, 210, 172, 91, 41; Anal. Calcd. for $C_{16}H_{23}NO_7S$:

Theory: C, 51.19; H, 6.71; N, 3.73; S, 8.54.

Found: C, 51.05; H, 6.50; N, 3.63; S, 8.13.

## Preparation 2

4-(R,S)-(t-Butoxycarbonylamino)-3-Oxo-1,2-Diazolidine

Under a nitrogen atmosphere, dry methylene chloride (50 ml) was cooled in an ice bath and anhydrous hydrazine (11.0 g, 333 mmole, 97%) was added. The ice bath was removed and the solution was stirred until it warmed to room temperature. At this time a solution of methyl 3-(p-toluenesulfonate)-2-(S)-(t-butoxycarbonylamino)propionate (20.0 g, 53.6 mmole) in dry methylene chloride (50 ml) was gradually added. The reaction solution was stirred under nitrogen at room temperature for 5 hours. The solution was then concentrated under reduced pressure and the concentrate was taken up in saturated aqueous sodium bicarbonate solution. The aqueous solution was continuously extracted for 14 hours with methylene chloride (700 ml). The methylene chloride solution was dried over sodium sulfate, filtered and concentrated under reduced pressure to yield approximately 5.15 g, 48% of 4-(R,S)-(t-butoxycarbonylamino)-3-oxo-1,2-diazolidine: n.m.r. ($CDCl_3$, 90 MHz): δ 7.04 (m, 1), 5.12 (m, 1), 4.28 (m, 1, C-4 proton), 3.94 (m, 1, C-5 proton), 3.20 (m, 1, C-5 proton), 1.45 (s, 9, t-butyl protons); i.r. ($CHCl_3$): 3430, 3250, 3019, 2983, 1702, 1545, 1503, 1370, 1297, 1241, 1215, 1165 $cm^{-1}$; f.d.m.s.: $M^+$ = 201;

Anal. Calcd. for $C_8H_{15}N_3O_3$:

Theory: C, 47.75; H, 7.51; N, 20.88.

Found: C, 47.80; H, 7.56; N, 20.61.

## Preparation 3

1-(p-Toluenesulfonyl)-2-(Allyl Carboxylate)-(E)-Ethylene

p-Toluenesulfinic acid sodium salt hydrate (2.03 g, 10.3 mmol) was dissolved in water (15 ml). Glacial acetic acid (0.57 ml, 10 mmol) and sodium acetate (0.82 g, 10 mmol) were added to the solution. A solution of allyl 2,3-dibromopropionate (2.72 g, 10.0 mmol) in dioxane (15 ml) was added to the reaction solution. The resultant emulsion was stirred at room temperature for 48 hours. The emulsion was diluted with methylene chloride and enough water to produce two phases. The organic phase was separated and washed with saturated aqueous sodium bicarbonate solution and brine, dried over sodium sulfate, filtered and concentrated under reduced pressure to yield 2.53 g, 95% of a colorless oil. The oil was stored overnight *in vacuo*, then

dissolved in a miniumum amount of ethanol and filtered. The filtrate was concentrated under reduced pressure to give 1-(1-p-toluenesulfonyl)-2-(allyl carboxylate)-(E)-ethylene, which was used without further purification: n.m.r. (90 MHz, $CDCl_3$) δ 7.76 (d, 2, J=9 aromatic protons), 7.34 (d, 2, J=9, aromatic protons), 7.32 (d, 1, J=16, olefinic proton), 6.78 (d, 1, J=16, olefinic proton), 6.12-5.68 (m, 1, allyl proton), 5.42-5.17 (m, 2, allyl protons), 4.66 (d, 2, J=6, allyl protons), 2.43 (s, 3, methyl protons).

## Preparation 4

### (Methylthio)(p-Toluenesulfonyl)Methane

Dimethylsulfoxide (9.25 g, 118 mmol) and acetic anhydride (15.6 g, 153 mmol) was heated to 80°C for 24 hours. The reaction solution was cooled to room temperature, then glacial acetic acid (90 ml), sodium acetate (9.7 g, 118 mmol) and sodium p-toluenesulfinate (31.6 g, 178 mmol, dried *in vacuo* overnight with $P_2O_5$ at 50°C) were added. The mixture was heated to 100°C for 24 hours. Brine (150 ml) was added and the solution was extracted with methylene chloride (5X, 100 ml). The combined organic extracts were washed with brine, dried over magnesium sulfate, filtered and concentrated *in vacuo* to give a gummy solid. The solid was recrystallized from 95% ethanol (3B) to yield 8.68 g of white crystals of (methylthio)(p-toluenesulfonyl)methane: m.p. 81-83°C.

## Preparation 5

t-Butyl 3-(R,S)-3-(Methylthio)-3-(p-Toluenesulfonyl)Propionate

(Methylthio)(p-toluenesulfonyl)methane (3.72 g, 17.2 mmol) was dissolved in THF (70 ml) and the solution was cooled to -78°C. n-Butyl lithium in hexane (11.5 ml. 17.2 mmol) was added and the solution was stirred at -78°C for 30 minutes. The solution was transferred via cannula to a stirred solution of t-butyl 2-bromoacetate (5.5 ml, 34.4 mmol) in THF (30 ml) at -78°C. The resultant solution was allowed to warm to room temperature, diluted with diethyl ether, washed with 1N hydrochloric acid and brine, dried over magnesium sulfate, filtered and concentrated *in vacuo* to an oil. The oil was diluted with toluene and concentrated *in vacuo*. The concentrate was reconstituted from hexanes containing a small amount of ether yield 3.33 g of colorless powder of t-butyl 3-(R,S)-3-(methylthio)-3-(p-toluenesulfonyl)propionate: n.m.r. (90 MHz, $CDCl_3$): δ 7.8 (d, 2, J = 8), 7.32 (d, 2, J = 8), 4.18 (dd, 1, J = 4, 11), 3.12 (dd, 1, J = 4, 16), 2.46 (dd, 1, J = 11, 16), 2.48 (s, 3), 2.26 (s, 3), 1.44 (s, 9); i.r. ($CHCl_3$): 1728 $cm^{-1}$; m.s.: $M^+$ = 330; Anal. Calcd. for $C_{15}H_{22}O_4S_2$:

Theory: C, 54.52; H, 6.71; S, 19.41.
Found: C, 54.78; H, 6.88; S, 19.68.

## Preparation 6

t-Butyl 3-(R,S)-3-Chloro-3-Methylthio-3-(p-Toluenesulfonyl)Propionate

t-Butyl 3-(R,S)-(3-methylthio)-3-(p-toluenesulfonyl)propionate (4.36 g, 13.12 mmol), sulfuryl chloride (1.1 ml, 13.5 mmol) and carbon tetrachloride (50 ml) were combined and stirred at room temperature for 16 hours. The reaction mixture was diluted with methylene chloride, then washed with saturated aqueous sodium bicarbonate solution and brine, dried over magnesium sulfate, filtered and concentrated in vacuo to give 4.16 g of yellow oil of t-butyl 3-(R,S)-3-chloro-3-methylthio-3-(p-toluenesulfonyl)propionate: n.m.r. (90 MHz, $CDCl_3$): δ 7.8 (m, 2), 7.3 (m, 2), 3.14 (s, 3), 2.5 (ABq, 2, J = 17), 2.5 (s, 3), 1.48 (s, 9).

## Preparation 7

t-Butyl 3-Methylthio-3-(p-Toluenesulfonyl)-acrylate

t-Butyl 3-(R,S)-3-Chloro-3-methylthio-3-(p-toluenesulfonyl)propionate (370 mg, 1 mmol) was dissolved in methylene chloride (5 ml) and the solution was cooled to -78°C. 1,8-Diazabicyclo[5.4.0]undec-7-ene (0.15 ml, 1 mmol) was added and the solution was stirred at -78°C for 15 minutes. The solution was warmed to room temperature, then diluted with diethyl ether, washed with 1N hydrochloric acid and saturated aqueous sodium

chloride solution, dried over magnesium sulfate, filtered and concentrated *in vacuo* to give 330 mg of yellow oil. The oil was chromatographed on a silica gel preparatory-scale TLC plate with an eluant of 4:1 hexane:ethyl acetate to give 230 mg of yellow oil (70%) of t-butyl 3-methylthio-3-(p-toluenesulfonyl)acrylate: n.m.r. ($CDCl_3$, 90 MHz): δ 7.8 (d, 2, J = 8), 7.3 (d, 2, J = 8), 7.18 (s, 1), 2.47 (s, 3), 2.40 (s, 3), 1.50 (s, 9); i.r. ($CHCl_3$): 1716, 1148 $cm^{-1}$; m.s.: $M^+$ = 328;
Anal. Calcd. for $C_{15}H_{20}O_4S_2$:
Theory: C, 54.85; H, 6.14.
Found: C, 54.70; H, 6.30.

## Preparation 8

4-(R,S)-(t-Butoxycarbonylamino)-3-Oxo-1-(Methylene)-1,2-Pyrazolidinium Ylide

4-(R,S)-t-(Butoxycarbonylamino)-3-oxo-1,2-diazolidine (4.02 g, 20 mmol) was dissolved in dry methanol (50 ml). 37% Aqueous formaldehyde (1.62 g, 20 mmol) was added, the mixture was stirred for 20 minutes at room temperature then concentrated *in vacuo*. The solvent was then azeotropically distilled several times with methanol *in vacuo* at 40°C. The resultant residue was dried *in vacuo* at 40°C overnight to yield 4-(R,S)-(t-butoxycarbonylamino)-3-oxo-1-(methylene)-1,2-pyrazolidinium ylide: n.m.r. (90 MHz, $CDCl_3$): δ 6.1-5.3 (m, 2), 4.9-4.2 (m, 6), 4.0-3.6 (m, 2), 3.5-3.1 (m, 2), 1.4 (s, 18); i.r. (KBr): 3379, 2980, 2930, 1705, 1524, 1519, 1504, 1455, 1393, 1368, 1297, 1252, 1166 $cm^{-1}$; f.d.m.s.: $M^{\oplus}$ = 213.

## Example 1

(2R,3S)- And (2S,3R)-2-(Allyl Carboxylate)-3-(p-Toluenesulfonyl)-7-(R,S)-(t-Butoxycarbonylamino)-8-Oxo-1,5-Diazabicyclo[3.3.0]Octane

4-(R,S)-(t-Butoxycarbonylamino)-3-oxo-1-methylene-1,2-pyrazolidinium ylide (1.92 g, 9.0 mmol), allyl 3-(p-toluenesulfonyl)acrylate (2.4 g, 9.0 mmol) and 1,2-dichloroethane (25 ml) were combined under nitrogen and refluxed for 48 hours. The reaction solution was concentrated under reduced pressure and chromatographed on a silica gel column eluted with a solvent gradient of 0 to 50% ethyl acetate in hexane. The chromatography yielded a mixture of 2R,3S and 2S,3R stereoisomers of 2-(allyl carboxylate)-3-(p-toluenesulfonyl)-7-(R,S)-(t-butoxycarbonylamino)-8-oxo-1,5-diazabicyclo[3.3.0]octane: n.m.r. (270 MHz, DMSO-$d_6$): δ 7.90-7.80 (m, 2), 7.58-7.16 (m, 2), 5.82-5.56 (m, 1), 5.28-5.12 (m, 2), 4.87-4.68 (m, 2), 4.66-4.28 (m, 3), 3.85-3.74 (m, 1), 3.66-3.46 (m, 1), 3.40-3.26 (m, 1), 3.20-3.08 (m, 0.5), 3.05-2.96 (m, 0.5), 2.88-2.72 (m, 1), 2.55 (s, 3), 1.39 (s, 9): i.r. ($CHCl_3$): 3018, 1711, 1502, 1369, 1326, 1306, 1291, 1284, 1215, and 1153 $cm^{-1}$; f.d.m.s.: $(M+2)^+ = 482$, $(M+1)^+ = 481$, $M^+ = 480$, $(M-1)^+ = 479$.

## Example 2

2-(R,S)-3-(R,S)-2-(t-Butyl Carboxylate)-3-Methylthio-3-(p-Toluenesulfonyl)-7-(R,S)-(t-Butoxycarbonylamino)-8-Oxo-1,5-Diazabicyclo[3.3.0]Octane

t-Butyl 3-methylthio-3-(p-toluenesulfonyl)-acrylate (1.7 g, 5.18 mmol), 4-(R,S)-(t-butoxycarbonylamino)-3-oxo-1-methylene-1,2-pyrazolidinium ylide (1.065 g, 5 mmol) and 1,2-dichloroethane (10 ml) were combined and refluxed for 24 hours. Additional pyrazolidinium ylide (570 mg, 2.67 mmol) was added and the solution was refluxed for an additional 24 hours. The solution was concentrated _in vacuo_ and was chromatographed by HPLC on a Waters Prep 500 silica gel column with a gradient elution of 0-10% ethyl acetate in hexane to give 650 mg of 2-(R,S)-3-(R,S)-2-(t-butyl carboxylate)-3-methylthio-3-(p-toluenesulfonyl)-7-(t-butoxycarbonylamino)-8-oxo-1,5-diazabicyclo[3.3.0]octane: n.m.r. (90 MHz, $CDCl_3$): δ 7.72 (m, 2), 7.24 (m, 2), 5.2-2.8 (m, 7), 2.54 (s, 3), 2.43 (br. s, 3), 1.46 and 1.43 (2 X s, 9), 1.35 (s, 9).

## Preparation 9

2-(t-Butyl Carboxylate)-3-(Methylthio)-7-(R,S)-(t-Butoxycarbonylamino)-8-Oxo-1,5-Diazabicyclo-[3.3.0]Octa-2-ene

2-(R,S)-3-(R,S)-2-(t-Butyl carboxylate)-3-methylthio-3-(p-toluenesulfonyl)-7-(R,S)-(t-butoxycarbonylamino)-8-oxo-5-diazabicyclo[3.3.0]octane (650 mg, 1.2 mmol) was dissolved in dichloromethane (2 ml) and the solution was cooled to -78°C. 1,8-Diazabicyclo[5.4.0]undec-7-ene (DBU) (195 microliters, 1.3 mmol) was added and the resultant solution was stirred at -78°C for 30 minutes, then warmed to room temperature. The solution was diluted with ethyl acetate and washed with 1N hydrochloric acid, saturated aqueous sodium bicarbonate solution and brine, dried over magnesium sulfate, filtered and concentrated _in vacuo_ to give 600 mg of residue. The residue was chromatographed on a preparatory-scale silica gel TLC plate eluted with a 1:1 mixture of hexane to ethyl acetate. The chromatography yielded 80 mg of 2-(t-butyl carboxylate)-3-(methylthio)-7-(R,S)-(t-butoxycarbonylamino)-8-oxo-1,5-diazabicyclo[3.3.0]-octa-2-ene: n.m.r. (90 MHz, $CDCl_3$): δ 5.32 (br. d, 1), 4.6 (m, 1), 4.34 (d, 1, J = 13), 4.04 (t, 1, J = 8), 3.72 (d, 1, J = 13), 2.68 (dd, 1, J = 8, 12), 2.31 (s, 3), 1.50 (s, 9), 1.40 (s, 9); u.v. (ethanol): $\lambda_{max}$ = 332 (ε = 3900); m.s.: $M^+$ = 385; i.r. ($CHCl_3$): 1710 $cm^{-1}$.

## Preparation 10

2-(Allyl Carboxylate)-7-(R,S)-(t-Butoxycarbonylamino)-8-Oxo-1,5-Diazabicyclo[3.3.0]Octa-2-ene

A mixture of 2R,3S and 2S,3R stereoisomers of 2-(allyl carboxylate)-3-(p-toluenesulfonyl)-7-(R,S)-(t-butoxycarbonylamino)-8-oxo-1,5-diazabicyclo[3.3.0]-octane (0.100 g, 0.209 mmol) was dissolved in dry

methylene chloride (5 ml) and the solution was cooled to -78°C. DBU (0.040 g, 0.263 mmol) was dissolved in dry methylene chloride (5 ml) and added to the cooled solution of bicyclic pyrazolidinone. The resultant reaction solution was stirred at -78°C for 1 hour, then warmed slowly to room temperature. Methylene chloride (15 ml) was added and the solution was washed with aqueous 0.1N hydrochloric acid (10 ml), saturated aqueous sodium bicarbonate solution (10 ml) and brine (10 ml), dried over magnesium sulfate, filtered and concentrated under reduced pressure to yield 0.080 g of 2-(allyl carboxylate)-7-(R,S)-(t-butoxycarbonylamino)-8-oxo-1,5-diazabicyclo[3.3.0]octa-2-ene: n.m.r. (90 MHz, $CDCl_3$): δ 6.26 (t, 1, J = 2.5), 6.1-5.6 (m, 1), 5.5-5.1 (m, 3), 4.9 (br. d, 2, J = 6), 4.58 (br. t, 1), 4.18 (dd, 1, J = 2.5, 14), 4.05 (t, 1, J = 7), 3.64 (dd, 1, J = 14, 2.5), 2.86 (dd, 1, J = 7, 12), 1.4 (s, 9); i.r. ($CHCl_3$): 1710 $cm^{-1}$; u.v. (95% ethanol): $\lambda_{max}$ = 311 (ε = 2150); m.s.: $M^+$ = 323.

## Preparation 11

2-(Allyl Carboxylate)-7-(R,S)-[2-(2-(Allyloxycarbonylamino)Thiazol-4-yl)-2-(Z)-Methoximinoacetamido)]-8-Oxo-1,5-Diazabicyclo[3.3.0]Octa-2-ene

A. Formation of the Side Chain Acid Chloride

DMF (0.136 ml, 1.6 mmol) was dissolved in ethyl acetate (4 ml) and the solution was cooled to 0°C. Phosphoryl chloride (0.102 ml, 1.1 mmol) was added

and the solution was stirred at 0°C for 1 hour. 2-(2-(allyloxycarbonyl)thiazol-4-yl)-2-(Z)-methoxyimino-acetic acid (314 mg, 1.1 mmol) was added and the solution was stirred for 1.5 hours at 0°C.

B. Removal of Amino Protecting Group and Formation of TFA Salt

2-(Allyl carboxylate)-7-(R,S)-(t-butoxycarbonylamino)-8-oxo-1,5-diazabicyclo[3.3.0]octa-2-ene (323 mg, 1 mmol) was dissolved in trifluoroacetic acid (3 ml). The solution was allowed to stand for 5 minutes then concentrated *in vacuo*. Methylene chloride (10 ml) was added to the concentrate and the resultant solution was concentrated *in vacuo*. Diethyl ether (15 ml) was added to the concentrate. The solution was sonicated and the diethyl ether was decanted to leave a yellow powder.

C. Neutralization of TFA Salt

The yellow powder from Step B was taken up in dry THF (5 ml) and BSTFA (1 ml) was added. The solution was stirred at 0°C for 30 minutes.

D. Acylation

The solutions from Steps A and C above were combined and stirred at 0°C for 2 hours. The solution was diluted with ethyl acetate, washed with 0.1 M hydrochloric acid, saturated aqueous sodium bicarbonate

solution and brine, dried over magnesium sulfate, filtered and concentrated in vacuo to give 350 mg of a brown solid. The solid was chromatographed on a preprepatory-scale TLC plate eluted with 10% methanol in ethyl acetate. The chromatography yielded 102 mg of 2-(allyl carboxylate)-7-(R,S)-[2-(2-(allyloxycarbonylamino)thiazol-4-yl)-2-(Z)-methoximinoacetamido)]-8-oxo-1,5-diazabicyclo[3.3.0]octa-2-ene: n.m.r. (90 MHz, $CDCl_3$) δ 8.4 (d, 1, J = 8), 7.1 (s, 1), 6.32 (br. s, 1), 6.2-5.6 (m, 2), 5.6-5.1 (m, 5), 4.7 (br. d, 5), 4.4-3.6 (m, 3), 3.88 (s, 3, superimposed on the absorption at 4.4-3.6), 3.02 (dd, 1, J = 8, 11); i.r. ($CHCl_3$): 1728, 1705, 1677, 1562 $cm^{-1}$; u.v. (95% ethanol): $\lambda_{max}$ = 310 (shoulder), 265 (ε = 13,400), 228 (ε = 18,200); m.s.: $M^+$ = 490.

## Preparation 12

2-(Carboxylic Acid)-7-(R,S)-[2-(2-Aminothiazol-4-yl)-2-(Z)-Methoximinoacetamido)]-8-Oxo-1,5-Diazabicyclo[3.3.0]Octa-2-ene

Triphenylphosphine (3 mg, 0.011 mmol) was dissolved in acetone (1 ml) then tetrakis[triphenylphosphine]palladium(0) (15 mg, 0.013 mmol) was added. 2-(Allyl carboxylate)-7-(R,S)-[2-(2-(allyloxycarbonyl)-thiazol-4-yl)-2-(Z)-methoximinoacetamido]-8-oxo-1,5-diazabicyclo[3.3.0]octa-2-ene (90 mg, 0.18 mmol) dissolved in acetone (3 ml) was added. The resultant solution was cooled to 0°C and tri(n-butyl)tin hydride

(0.103 ml, 0.38 mmol) was added. The solution was stirred at room temperature for 1 hour then cooled again to 0°C. 1N Hydrochloric acid (0.38 ml) was added and the solution was stirred for 10 minutes. Water (10 ml), followed by a small amount of acetonitrile, was added and the solution was extracted with hexane (3X, 40 ml). The aqueous phase was freeze-dried after the acetone and acetonitrile were removed *in vacuo*. The freeze-drying procedure yielded 80 mg of crude yellow powder. The powder was taken up in water, filtered, and the filtrate was freeze-dried to yield 72 mg of 2-(carboxylic acid)-7-(R,S)-[2-(2-aminothiazol-4-yl)-2-(Z)-methoximinoacetamido)]-8-oxo-1,5-diazabicycli[3.3.0]octa-2-ene: n.m.r. (90 MHz, $D_2O$): δ 7.5 (m, 2), 7.0 (s, 1), 6.1 (m, 1), 5.2-4.8 (m, 2), 4.1-3.5 (m, 3), 3.84 (s, 3, superimposed on the absorbance at 4.1-3.5), 3.04 (dd, 1, J = 9, 12).

## Preparation 13

## N-(t-Butoxycaronyl) (L)-Serine Trifluoroacetyl Acyl Hydrazide

N-(t-Butoxycarbonyl) (L)-serine acyl hydrazide (32.85 g, 150 mmol) was suspended in ethanol (400 ml). Ethylthio trifluorothioacetate (30 ml, 37.02 g, 234.3 mmol) was added to the suspension and the resultant mixture was stirred at room temperature for 65 hours. The solvent was removed *in vacuo* and the residue was dissolved in diethyl ether (160 ml). A seed crystal was added to the diethyl ether solution and the resultant

crystals were collected by filtration (approx. 27 g). The filtrate was evaporated *in vacuo* and diethyl ether (50 ml) was added to the residue. The solids that formed on standing were removed by filtration to yield approx. 16.5 g of additional product. The two batches of solids collected by filtration were combined and recrystallized from diethyl ether (3 liters). After affecting solution, the solution was reduced to approximately 450 ml to yield (after a second crop) 41.04 g, 87% yield of N-(t-butoxycarbonyl) (L)-serine trifluoroacetyl acyl hydrazide: n.m.r. (300 MHz, DMSO-$d_6$): $\delta$ 11.5 (br. s, 1), 10.33 (s, 1), 6.84 (d, 1, J=9), 4.9 (t, 1, J=7, (OH), 4.1 (m, 1), 3.59 (br. m, 2), 1.4 (s, 9); specific rotation: $[\alpha]_D^{25}$ = -25.87° (10.05 mg/ml, methanol); m.p.: 143-144°C (first crop), 142°-144°C (second crop).

Anal. Calcd for $C_{10}H_{16}N_3O_5F_3$:

Theory: C, 38.10; H, 5.12; N, 13.33;

Found: C, 38.34; H, 4.89; N, 13.16

## Preparation 14

### 4-(S)-(t-Butoxycarbonylamino)-1-(Trifluoroacetyl)-3-Oxo-1,2-Diazolidine

N-(t-Butoxycarbonyl) (L)-serine trifluoroacetyl acyl hydrazide (3.78 g, 12 mmol) and triphenylphosphine (3.46 g, 13.2 mmol) were dissolved in THF (50 ml). To the solution was added a THF solution (10 ml) of 95% diethyl azodicarboxylate (2.42 g, 2.19 ml, 13.2

mmol). The resultant mixture was stirred at room temperature for six hours and then the solvent was removed *in vacuo*. The residue was dissolved in ethyl acetate (100 ml) and then the solution was washed with aqueous sodium bicarbonate solution (33 ml, 3X). The sodium bicarbonate extracts were combined, aqueous saturated brine solution (70 ml) was added and the resultant mixture was extracted with ethyl acetate (120 ml, 3X). The sodium bicarbonate solution was then layered with additional ethyl acetate (200 ml) and 1N hydrochloric acid (approx. 80 ml) was added until the sodium bicarbonate solution had a pH of 2.5. The ethyl acetate layer was separated and the aqueous layer was extracted with additional ethyl acetate (4X, 125 ml). The ethyl acetate extracts were combined, washed with saturated aqueous brine (125 ml, 2X), dried over sodium sulfate, filtered, and taken to dryness *in vacuo*. The resultant residue was dissolved in acetonitrile (100 ml) and the acetonitrile was removed *in vacuo*. Treatment of the residue with acetonitrile was repeated to yield 3.06 g, 96% yield of 4-(S)-(t-butoxycarbonylamino)-1-(trifluoroacetyl)-3-oxo-1,2-diazolidine: n.m.r. (300 MHz, $CDCl_3$): δ 5.25 (d, 1, J=6), 4.81 (t, 1), 4.6 (m, 1), 4.06 (t, 1), 1.46 (s, 9); i.r. ($CHCl_3$): 1722, 1682, 1518 $cm^{-1}$; f.d.m.s. (m/e): $M^+$ = 297; specific rotation: $[\alpha]_D^{25}$ = -88.14° (10.03 mg/ml in methanol);

Anal. Calcd for $C_{10}H_{14}N_3O_4F_3$: C, 40.41; H, 4.75; N, 14.14.

Found: C, 40.58; H, 5.01; N, 13.92.

## Preparation 15

### 4-(S)-(t-Butoxycarbonylamino)-3-Oxo-1,2-Diazolidine

4-(S)-(t-butoxycarbonylamino)-1-(trifluoroacetyl)3-oxo-1,2-diazolidine (2.97 g, 10 mmol) was suspended in water (30 ml), 1 N sodium hydroxide solution (20 ml, 0.8 g, 20 mmol, pH 12.2) was added and the resultant mixture was stirred for one hour at room temperature. The pH of the mixture was adjusted to 7.2 by the addition of 1N hydrochloric acid (10 ml). Sodium chloride (13 g) was added to the solution and the mixture was extracted with chloroform (50 ml, 8X). The chloroform extracts were combined, washed with saturated aqueous sodium chloride solution (75 ml), dried over sodium sulfate, filtered, and evaporated to dryness *in vacuo*. Diethyl ether (100 ml) was added to the residue and the ether was removed *in vacuo* to yield 0.798 g of a white solid of 4-(S)-(t-butoxycarbonylamino)-3-oxo-1,2-diazolidine: n.m.r. (300 MHz, DMSO-$d_6$): δ 9.23 (s, 1), 7.04 (d, 1, J=9), 5.24 (br. s, 1,), 4.24 (m, 1), 3.41 (t, 1), 2.88 (t, 1), 1.38 (s, 9); specific rotation: $[\alpha]_D^{25°}$ = - 74.16° (10.06 mg/ml in methanol); (the compound was dried overnight at 80°C before analysis):

Anal. Calcd for $C_8H_{15}N_3O_3$: C, 47.75; H, 7.51; N, 20.88.
Found: C, 47.75; H, 7.46; N, 20.62.

Preparation 16

(Phenylsulfonyl)acetone

Sodium benzenesulfinate (50 g, 304.6 mmol) was dissolved in water (200 ml) and chloroacetone (29.6 g, 319.9 mmol) then dioxane (200 ml) were added to the solution. The solution was stirred and heated to 50°C for 22 hours and ice was added to cool the reaction solution to approximately 25°C. The cooled solution was extracted with methylene chloride (2X, 250 ml), the methylene chloride layers were combined and washed with saturated aqueous sodium bicarbonate solution (1X, 250 ml) and water (1X, 250 ml) then the aqueous wash solutions were combined and extracted with methylene chloride (2X, 100 ml). All of the methylene chloride layers were combined and washed with saturated aqueous sodium chloride solution (1X, 150 ml), dried over sodium sulfate, filtered and concentrated *in vacuo* to a purple solid. The solid was dissolved in diethyl ether (800 ml) with gentle heating and then the solution was stirred and allowed to cool to room temperature. The solution was slowly cooled to 0°C with stirring, causing the product to crystallize. The resulting crystals were collected by filtration, washed with cold diethyl ether, and dried *in vacuo* at 35°C for 4 hours to yield 36.43 g of (phenylsulfonyl)acetone. The filtrate was concentrated to approximately one-eighth of its volume *in vacuo* and was gradually cooled below room temperature to induce crystallization, then stirred overnight at room

temperature. The resulting crystals were collected by filtration, washed with cold diethyl ether, and dried *in vacuo* at 35°C for 8 hours to yield an additional 14 g of the (phenylsulfonyl)acetone: n.m.r. (90 MHz, $CDCl_3$): δ 2.39 (s, 3), 4.17 (s, 2), 7.55-7.95 (m, 5); m.p. 56-58°C.

## Preparation 17

## Allyl 3-(Phenylsulfonyl)-4-Oxopentanoate

Under a nitrogen atmosphere, sodium hydride (10.7 g, 0.267 mol) and THF (125 ml) were combined and the suspension was cooled to -15°C. A THF solution (150 ml) of (phenylsulfonyl)acetone (50 g, 0.253 mol) was added to the suspension in a dropwise fashion over an hour. The resultant mixture was stirred for an additional 20 minutes at -15°C. Allyl bromoacetate (47.5 g, 0.265 mol) dissolved in THF (100 ml) was added in a dropwise fashion to the mixture over a period of 30 minutes while maintaining the temperature of the solution below 0°C. The resultant solution was stirred at -5°C for 15 minutes, allowed to warm to room temperature over a period of an hour, and stirred overnight at room temperature. The reaction solution was poured into saturated aqueous ammonium chloride solution (800 ml) and chloroform (500 ml) and the aqueous layer was separated and extracted with chloroform (2X, 200 ml). The chloroform layers were combined and washed with saturated aqueous sodium chloride (1X, 350 ml), dried

over sodium sulfate, filtered and concentrated in vacuo to an oil. The oil was recrystallized from a mixture of ethyl acetate and hexane. The collected solid was washed with cold 3:1 ethyl acetate: hexane and dried in vacuo, yielding 52.9 g of the allyl 3-(phenylsulfonyl)-4-oxopentanoate: n.m.r. (90 MHz, $CDCl_3$): δ 2.51 (s, 3), 2.97 (n, 2,), 4.51 (dm, 2, J=6), 4.65 (m, 1), 5.1-5.19 (m,2), 5.58-6.00 (m, 1), 7.4-7.8 (m, 5).

## Preparation 18

### Allyl 3-(Phenylsulfonyl)-4-Oxopent-2-(E)-Enoate

Allyl 3-(phenylsulfonyl)-4-oxopentanoate (42 g, 141.9 mmol) was combined with chloroform (400 ml). Upon dissolution the solution was cooled to -20°C and then triethylamine (39.4 ml, 282.8 mmol) was added while maintaining the temperature between -25°C to -20°C. A chloroform solution of bromine (238 ml, 0.594M, 141.4 mmol) was added in a dropwise fashion over a 1 hour period. The solution was stirred at -20°C for 30 minutes then at room temperature overnight. The reaction mixture was washed with water (2X, 200 ml), 0.5N hydrochloric acid (1X, 200 ml), saturated aqueous sodium bicarbonate solution (1X, 200 ml), saturated sodium chloride solution (1X, 250 ml), dried over sodium sulfate, filtered and and concentrated in vacuo to yield 41.76 g of allyl 3-(phenylsulfonyl)-4-oxopent-2-(E)-enoate as an oil: n.m.r. (90 MHz, $CDCl_3$): δ 2.50 (s, 3), 4.65 (dm, J = 6), 5.20 - 5.44 (m, 2), 5.65-6.10, (m, 1), 6.86 (s, 1), 7.42-7.85 (m, 5); m.p. 150°C.

## Example 3

### 2-(Allyl Carboxylate)-3-(Acetyl)-7-(S)-(t-Butoxycarbonylamino)-8-Oxo-1,5-Diazabicyclo[3.3.0]Octa-2-ene

### Step A

### 4-(S)-(t-Butoxycarbonylamino)-3-Oxo-1-Methylene-1,2-Pyridazolinium Ylide

4-(S)-(t-Butoxycarbonylamino)-3-oxo-1,2-diazolidine (22.7 g, 113 mmol), formalin (37%, 10.1 g, 124.6 mmol) and 1, 2-dichloroethane (450 ml) were combined and stirred at room temperature for 2 hours. The solution was evaporated *in vacuo* to a solid that was taken up in toluene (200 ml) and concentrated again *in vacuo*.

### Step B

### Cycloaddition

Allyl 3-(phenylsulfonyl)-4-oxopent-2-(E)-enoate (40 g, 136 mmol) was washed into a flask with 1,2- dichloroethane (150 ml). The solution was heated to 70°C and then a 1,2-dichloroethane solution (300 ml) of the ylide of Step A was added over a period of two hours while maintaining the temperature of the solution between 70° to 75°C. The solution was heated to reflux then allowed to cool to 24°C. The solution contained 2-(R,S)-(allyl carboxylate)-3-(R,S)-acetyl-3-(R,S)-phenylsulfonyl)-7-(S)-(t-butoxycarbonylamino)-8-oxo-1,5-diazabicyclo[3.3.0]octane.

## Step C

## Elimination

N-methylmorpholine (12.5 ml, 113.6 mmol) was added to the solution of Step B over a period of one minute, and the resultant solution was stirred overnight at room temperature. The solvent was evaporated in vacuo and the resultant oily product was taken up in ethyl acetate (1 liter). The solution was washed with 0.2N hydrochloric acid (2X, 250 ml), water (1X, 250 ml), saturated aqueous sodium bicarbonate solution (1X, 250 ml) and saturated aqueous sodium chloride solution (1X, 250 ml), dried over sodium sulfate, filtered and concentrated in vacuo to a volume of approximately 165 ml. Additional ethyl acetate (50 ml) was added and the solution was heated until all solids dissolved. Hexane (100 ml) was slowly added, the solution was first stirred at room temperature for 1.5 hours then at 0°C for one hour and filtered. The collected solid was washed with a 0°C mixture of 30% ethyl acetate/70% hexane (2X, 100 ml) to yield a yellow crystalline solid. The solid was dried in vacuo at 40°C for 3 hours to give 25.3 g, 61.4% of the 2-(allyl carboxylate)-3-(acetyl)-7-(S)-(t-butoxycarbonylamino)-8-oxo-1,5-diazabicyclo-[3.3.0]octa-2-ene: n.m.r. (90 MHz, $CDCl_3$): δ 1.45 (s, 9), 2.27 (s, 3), 2.83 (dd, J=11 and 8, 1), 4.02 (br. t, 1), 3.91 (d, J=12.5,1), 4.39 (d, J=12.5,1) 4.68 (m, 1), 4.87 (dm, 2, J=6), 5.15 (br. d, 1), 5.3-5.5 (m, 2), 5.8-6.2 (m, 1); i.r. ($CHCl_3$): 3440, 1750, 1717 $cm^{-1}$; u.v. (methanol): $\lambda_{max}$ = 225 ($\varepsilon_{max}$ = 8,927), 364 (8667); f.d.m.s. (m/e): $M^+$ = 365; m.p. 136-137°C; $[\alpha]_D^{25}$ = -644° (c 1, methanol).

## Preparation 19

### 2-(Allyl Carboxylate)-3-Acetyl-7-(S)-[2-(2-(Allyloxycarbonylamino)thiazol-4-yl)-2-(Z)-Methoximinoacetamido]-8-Oxo-1,5-Diazabicyclo[3.3.0]Octa-2-ene

### Step A

### Formation of Acid Chloride

Under a nitrogen atmosphere, ethyl acetate (55 ml) and DMF (2.2 ml, 28.4 mmol) were combined and cooled to 0°C. Phosphoryl chloride (1.8 ml, 19.3 mmol) was added and the solution was stirred at 0°C for 75 minutes. 2-(2-(N-allyloxycarbonylamino)thiazol-4-yl)-2-(Z)-methoxyiminoacetic acid (5.5 g, 19.3 mmol) was added and the solution was stirred at 0°C for two hours.

### Step B

### Synthesis of 2-(Allyl Carboxylate)-3-Acetyl-7-(S)-Amino-1,5-Diazabicyclo[3.3.0]Octa-2-ene Hydrochloride

2-(Allyl carboxylate)-3-(acetyl)-7-(S)-(t-butoxycarbonylamino)-8-oxo-1,5-diazabicycl[3.3.0]octa-2-ene (7.0 g, 19.2 mmol) was stirred with 3N hydrogen chloride in acetic acid (50 ml) for twenty minutes at room temperature. The acetic acid was removed *in vacuo*, and methylene chloride was added to the residue and the mixture evaporated *in vacuo*. Methylene chloride was again added and the solution taken to dryness *in vacuo*, affording a thick, orange oil.

## Step C

## Acylation

The hydrochloride nucleus from Step B above was dissolved in a mixture of acetonitrile (250 ml) and water (250 ml) and the solution was cooled to 5°C. The pH of the solution was raised from 1.9 to 7.25 by the addition of 1M $K_2HPO_4$ solution. The solution containing the acid chloride from Step A above was added to this cooled solution over a period of 15 minutes while maintaining the temperature of the solution below 5°C and the pH of the solution at approximately 7. The resultant solution was stirred for 30 minutes at 3°C then at room temperature for 1.5 hours. Ethyl acetate (250 ml) was added, the aqueous layer was separated and extracted with additional ethyl acetate (1X, 250 ml). The ethyl acetate layers were combined, washed with water (1X, 250 ml), saturated aqueous sodium bicarbonate solution (1X, 250 ml), water (1X, 250 ml), saturated sodium chloride solution (1X, 250 ml), dried over sodium sulfate, filtered and evaporated to dryness _in vacuo_ to give 6.96 g, 68% yield of a yellow solid of the 2-(allyl carboxylate)-3-acetyl-7-(S)-[2-(2-(allyloxycarbonylamino)-thiazol-4-yl)-2-(Z)-methoximinoacetamido]-8-oxo-1,5-diazabicyclo[3.3.0]octa-2-ene: n.m.r. (90 MHz, $CDCl_3$): δ 2.28 (s, 3), 3.0 (m, 1), 3.95 (s, 3), 3.96 (d, J=12.5, 1), 4.08 (t, 1, partially observed), 4.39 (d, 5 = 12.5, 1), 4.69 (dm, 2, J=5.4), 4.78 (d, 2, J=3.6), 5.28-5.44 (m, 4), 5.7-6.2 (2), 7.14 (s, 1), 8.18 (br. d, 1).

## Preparation 20

### 2-(Carboxylic Acid)-3-Acetyl-7-(S)-[2-(2-Aminothiazol-4-yl)-2-(Z)-Methoximinoacetamido]-8-Oxo-1,5-Diazabicyclo [3.3.0]Octa-2-ene

Under a nitrogen atmosphere, palladium(II) acetate (26 mg) was combined with acetone (5 ml) then triphenylphosphine (131 mg) was washed into the solution with acetone (5 ml). The solution was stirred for 10 minutes at room temperature then 2-(allyl carboxylate)-3-acetyl-7-(S)-[2-(2-(allyloxycarbonylamino)thiazol-4-yl)-2-(Z)-methoximinoacetamido]-8-oxo-1,5-diazabicyclo-[3.3.0]octa-2-ene (0.8 g) as an acetone solution (20 ml) was added. The resultant solution was stirred first for 40 minutes at room temperature then cooled to 2°C. Tri(n-butyl)tin hydride (0.81 ml) was added and the solution was stirred at 0°C for 30 minutes then at room temperature for approximately two hours. The solution was cooled to approximately 2°C and 1N hydrochloric acid (3.0 ml) was added. The cooling bath was removed and the solution was stirred for 10 minutes then filtered. Water (200 ml) was added to the filtrate. Acetonitrile (40 ml) was added to redissolve the orange oil that formed. The resulting solution was washed with hexane (2X, 50 ml), filtered through Hy-Flo™ and the resultant filtrate was washed with diethyl ether (2X, 50 ml) and hexane (1X, 50 ml), concentrated in vacuo at 40°C then lyophilized. The lyophilized material was chromatographed on a preparatory-scale high performance liquid

chromatography apparatus using a reverse phase C-18 column eluted with a gradient of 0% to 20% methanol in water. The product-containing fractions were combined and lyophilized to give 0.155 g of the 2-(carboxylic acid)-3-acetyl-7-(S)-[2-(2-aminothiazol-4-yl)-2-(Z)-methoximinoacetamido]-8-oxo-1,5-diazabicyclo-[3.3.0]octa-2-ene: n.m.r.(90 MHz, $D_2O$): δ 2.34 (s, 3), 3.23 (dd, 1, J=11 and 9), 3.97 (s, 3), 3.84-4.10 (m, 2), 4.29 (d, J= 12.5, 1), 5.21 (dd, 1, J=13.5 and 8), 7.06 (s, 1); specific rotation $[\alpha]_D^{25}$ = -347° (c = 0.4 methanol).

## Claims

1. A compound of Formula I:

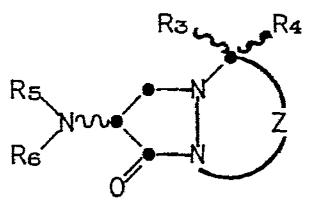

(I)

wherein Z is a group of the formula

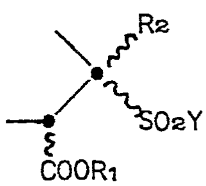

or

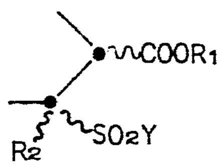

,

and wherein:

$R_1$ is hydrogen, an organic or inorganic cation, a carboxy-protecting group or a non-toxic, metabolically-labile, ester-forming group;

$R_2$ is hydrogen, halo, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano;

a group of the formula

$-CX_3$

wherein X is fluoro, chloro, bromo or iodo;

a group of the formula

$$-\overset{(O)_z}{\overset{\|}{S}}-R_7$$

wherein z is 0, 1 or 2 and $R_7$ is $C_1$ to $C_6$ alkyl, $C_1$ to $C_6$ substituted alkyl, phenyl, substituted phenyl, $C_7$ to $C_{12}$ arylalkyl , $C_7$ to $C_{12}$ substituted aryl-alkyl, or a heterocyclic ring;

a group of the formula

$$-COR_8$$

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted aryl-alkyl, phenyl, substituted phenyl, amino, (monosubstituted)amino or disubstituted amino;

a group of the formula

$$-COOR_9$$

wherein $R_9$ is hydrogen, an organic or inorganic cation, $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl, a carboxy-protecting group or a non-toxic, metabolically-labile, ester-forming group;

or a group of the formula

$$-CH_2-S-\text{Heterocyclic ring};$$

$R_3$ and $R_4$ are hydrogen or; either $R_3$ or $R_4$ is $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl or a group of the formula

$$-COOR_{10}$$

wherein $R_{10}$ has the same definition as $R_9$; and the other is hydrogen;

$R_5$ and $R_6$ are

1) each hydrogen; or

2) taken together and form a phthalimido group; or

3) either $R_5$ or $R_6$ is hydrogen and the other is an amino-protecting group; and

Y is $C_1$ to $C_6$ alkyl, $C_1$ to $C_6$ substituted alkyl, phenyl, substituted phenyl, $C_7$ to $C_{12}$ arylalkyl or $C_7$ to $C_{12}$ substituted arylalkyl;

or a pharmaceutically acceptable salt thereof.

2. A compound according to claim 1, wherein either $R_5$ or $R_6$ is hydrogen and the other of $R_5$ or $R_6$ is an amino-protecting group.

3. A compound according to claim 1 or 2, wherein $R_2$ is:

a) hydrogen, halo, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano; or

b) a group of the formula

$$-\overset{(O)_z}{\overset{||}{S}}-R_7 \quad .$$

4. A compound according to any one of claims 1 to 3, wherein Y is phenyl or substituted phenyl and $R_3$ and $R_4$ are the same and are hydrogen.

5. A compound according to any one of claims 1 to 4, wherein Z is a group of the formula

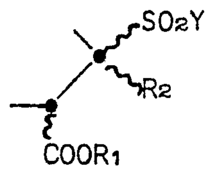

6. A compound according to any one of claims 1 to 5 of the following formula

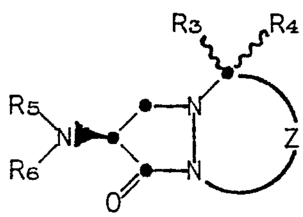

7. A process for preparing a compound of Formula I as claimed in any one of claims 1 to 6, which comprises reacting an ylide of Formula A

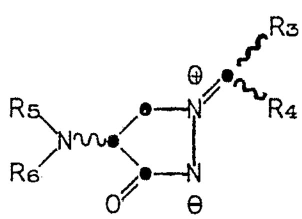

(A)

with an ethylene of Formula (B)

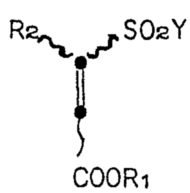

(B)

8. A process according to Claim 7 for preparing a compound of Formula I wherein the ylide is of the formula:

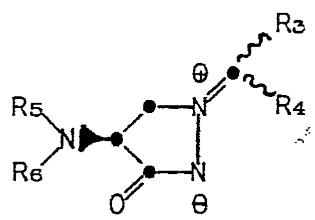

9. A process according to Claim 7 or 8 for preparing a compound of Formula I wherein the ethylene used is of the formula:

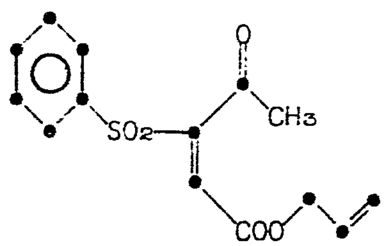

Claims

1. A process for preparing a compound of Formula I:

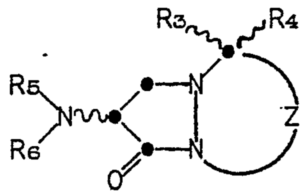

(I)

wherein Z is a group of the formula

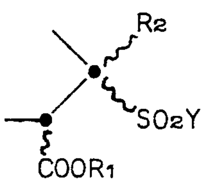

or

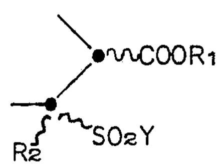

,

and wherein:

$R_1$ is hydrogen, an organic or inorganic cation, a carboxy-protecting group or a non-toxic, metabolically-labile, ester-forming group;

$R_2$ is hydrogen, halo, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano;

a group of the formula

$-CX_3$

wherein X is fluoro, chloro, bromo or iodo;

a group of the formula

$$-\overset{(O)_z}{\overset{\|}{S}}-R_7$$

wherein z is 0, 1 or 2 and $R_7$ is $C_1$ to $C_6$ alkyl, $C_1$ to $C_6$ substituted alkyl, phenyl, substituted phenyl, $C_7$ to $C_{12}$ arylalkyl , $C_7$ to $C_{12}$ substituted arylalkyl, or a heterocyclic ring;

a group of the formula

$$-COR_8$$

wherein $R_8$ is hydrogen, $C_1$ to $C_6$ alkyl, $C_1$ to $C_6$ substituted alkyl, perfluoro $C_2$ to $C_4$ alkyl, trihalomethyl, $C_7$ to $C_{12}$ arylalkyl, $C_7$ to $C_{12}$ substituted arylalkyl, phenyl, substituted phenyl, amino, (monosubstituted)amino or disubstituted amino;

a group of the formula

$$-COOR_9$$

wherein $R_9$ is hydrogen, an organic or inorganic cation, $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl, a carboxy-protecting group or a non-toxic, metabolically-labile, ester-forming group;

or a group of the formula

$$-CH_2-S\text{-Heterocyclic ring;}$$

$R_3$ and $R_4$ are hydrogen or; either $R_3$ or $R_4$ is $C_1$ to $C_6$ alkyl, $C_1$ to $C_6$ substituted alkyl, $C_7$ to $C_{12}$ arylalkyl, $C_7$ to $C_{12}$ substituted arylalkyl, phenyl, substituted phenyl or a group of the formula

$$-COOR_{10}$$

wherein $R_{10}$ has the same definition as $R_9$; and the other is hydrogen;

$R_5$ and $R_6$ are

1) each hydrogen; or
2) taken together and form a phthalimido group; or
3) either $R_5$ or $R_6$ is hydrogen and the other is an amino-protecting group; and

Y is $C_1$ to $C_6$ alkyl, $C_1$ to $C_6$ substituted alkyl, phenyl, substituted phenyl, $C_7$ to $C_{12}$ arylalkyl or $C_7$ to $C_{12}$ substituted arylalkyl;

or a pharmaceutically acceptable salt thereof, which comprises reacting an ylide of Formula A

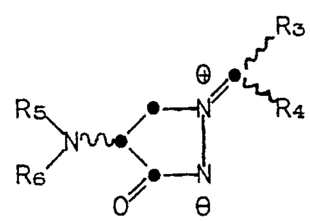

(A)

with an ethylene of Formula (B)

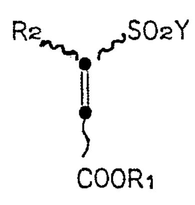

(B)

2. A process according to Claim 1 for preparing a compound of Formula I wherein the ylide is of the formula:

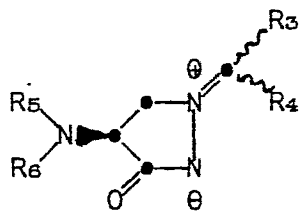

.

3. A process according to claim 1 or 2, wherein either $R_5$ or $R_6$ is hydrogen and the other of $R_5$ or $R_6$ is an amino-protecting group.

4. A process according to claim 1, 2, or 3, wherein $R_2$ is:

a) hydrogen, halo, $C_1$ to $C_6$ alkyl, $C_1$ to $C_6$ substituted alkyl, perfluoro $C_2$ to $C_4$ alkyl, $C_7$ to $C_{12}$ arylalkyl, $C_7$ to $C_{12}$ substituted arylalkyl, phenyl, substituted phenyl or cyano; or

b) a group of the formula

$$\overset{(O)_z}{\overset{\|}{-S-R_7}} \quad .$$

5. A process according to any one of claims 1 to 4, wherein Y is phenyl or substituted phenyl and $R_3$ and $R_4$ are the same and are hydrogen.

6. A process according to any one of claims 1 to 5, wherein Z is a group of the formula

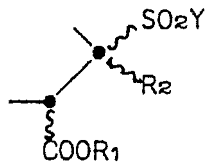

.

7. A process according to Claim 6 for preparing a compound of Formula I wherein the ethylene used is of the formula:

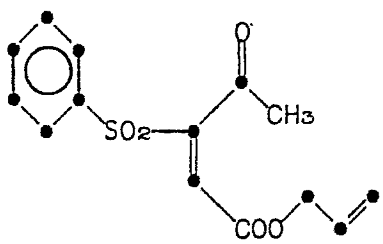

.

European Patent Office

**0202794**

# EUROPEAN SEARCH REPORT

Application number

EP 86 30 3175

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | CHEMICAL ABSTRACTS, vol. 95, no. 5, 3rd August 1981, page 761, abstract no. 43100m, Columbus, Ohio, US; & DD - A - 143 617 (AKADEMIE DER WISSENSCHAFTEN DER DDR) 03-09-1980 * Abstract * --- | 1,7 | C 07 D 487/04 //<br>C 07 D 231/08<br>(C 07 D 487/04<br>C 07 D 231:00<br>C 07 D 231:00 ) |
| A | TETRAHEDRON, vol. 24, no. 23, December 1968, pages 6809-6811, Pergamon Press, GB; H. DORN et al.: "Stabile Azomethinimine und 1-substituierte 3-hydroxy-pyrazole durch Dehydrierung 1-substituierter Pyrazolidone-(3)" * Whole document * --- | 1,7 | |
| A | US-A-4 128 425 (R.B. GREENWALD) * Column 4, line 45; columns 23-25 * ----- | 1 | TECHNICAL FIELDS SEARCHED (Int Cl 4)<br>C 07 D 487/00 |

The present search report has been drawn up for all claims

| Place of search | Date of completion of the search | Examiner |
|---|---|---|
| THE HAGUE | 11-08-1986 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82